# EUROPEAN PATENT APPLICATION

(11) **EP 4 279 085 A1**
(43) Date of publication of application: **22.11.2023**
(21) Application number: 22305760.5
(22) Date of filing: 20.05.2022
(51) Int. Cl.: A61K 39/00, C07K 14/725, C12N 15/113

(54) **COMPOSITIONS AND METHODS FOR TREATING A REFRACTORY OR RELAPSED CANCER OR A CHRONIC INFECTIOUS DISEASE**

(71) Applicant: Mnemo Therapeutics, 75016 Paris (FR); Institut Curie, 75005 Paris (FR); Centre National de la Recherche Scientifique (CNRS), 75016 Paris (FR)
(72) Inventor: The designation of the inventor has not yet been filed
(74) Representative: Plasseraud IP

(57) **Abstract**

The invention provides compositions and methods for treating a refractory, relapsed or resistant cancer or a chronic infectious disease. In particular the present invention relates to a modified immune cell with reduced SUV39H1 activity, for use in the treatment of a patient suffering from a refractory, relapsed or resistant cancer or suffering from a chronic infectious disease, wherein the cell expresses one or more engineered antigen-specific receptors that bind an antigen associated with the cancer or the chronic infectious disease

## Description

### FIELD OF THE INVENTION

The invention relates to compositions and methods for treating a refractory, relapsed or resistant cancer or a chronic infectious disease.

### BACKGROUND OF THE INVENTION

A variety of cancer therapies, including chemotherapy, antibody therapy, and adoptive cell therapy, are widely available. Yet they encounter hurdles such as cancers that are refractory or resistant to treatment, or cancers that relapse after an initial successful treatment.

Cell immunotherapy has been widely researched and several CAR T-cell therapies are commercially marketed for the treatment of B-cell or lymphoid malignancies. However, despite the impressive rates of initial complete responses observed with current CAR T-cell therapies for B-cell malignancies, longer term follow-up has demonstrated that a significant proportion of patients relapse after treatment. In addition, clinically effective treatment of solid tumors and myeloid malignancies has proven more challenging than treatment of lymphoid malignancies.

There remains a need for better or more effective treatments for cancer.

### SUMMARY OF THE INVENTION

The disclosure provides a modified immune cell with reduced SUV39H1 activity, for use in the treatment of a patient suffering from a refractory, relapsed or resistant cancer or suffering from a chronic infectious disease. Optionally, the cell expresses one or more engineered antigen-specific receptors that bind an antigen associated with the cancer or the chronic infectious disease. In related aspects, the disclosure provides for use of such modified immune cells for preparation of a medicament for treating a patient suffering from a refractory, relapsed or resistant cancer or suffering from a chronic infectious disease. In further related aspects, the disclosure provides for methods of treating a patient suffering from a refractory, relapsed or resistant cancer or suffering from a chronic infectious disease by administering a modified immune cell with reduced SUV39H1 activity, optionally comprising one or more engineered antigen-specific receptors that bind an antigen associated with the cancer or the chronic infectious disease.

In some embodiments, when the patient suffers from a cancer, the patient exhibits a cancer relapse or is likely to exhibit a cancer relapse, exhibits cancer metastasis or is likely to exhibit cancer metastasis, has not achieved sustained cancer remission after one or more prior cancer therapies, suffers from a cancer that is resistant or nonresponsive to one or more prior cancer therapies, suffers from a refractory cancer, is likely to exhibit a response to cell therapy that is not durable, is ineligible for immune checkpoint therapy or did not respond to immune checkpoint therapy, is ineligible for treatment with high dose of chemotherapy and/or is ineligible for treatment with high adoptive cell therapy doses.

In some embodiments, the antigen is orphan tyrosine kinase receptor ROR1, tEGFR, Her2, p95HER2, Ll-CAM, CD19, CD20, CD22, mesothelin, CEA, Claudin 18.2, hepatitis B surface antigen, anti-folate receptor, a claudin (such as claudin 6 or claudin 18.2), CD23, CD24, CD30, CD33, CD38, CD44, EGFR, EGP-2, EGP-4, CD70, EPHa2, ErbB2, 3, or 4, FcRH5, FBP, fetal acethycholine e receptor, GD2, GD3, HMW-MAA, IL-22R-alpha, IL-13R-alpha2, kdr, kappa light chain, BCMA, Lewis Y, MAGE-A1, mesothelin, MUC1, MUC16, PSCA, NKG2D Ligands, NY-ESO-1, MART-1, gplOO, oncofetal antigen, TAG72, VEGF-R2, carcinoembryonic antigen (CEA), prostate specific antigen (PSMA), estrogen receptor, progesterone receptor, ephrinB2, CD 123, CS-1, c-Met, GD-2, MAGE A3, CE7, or Wilms Tumor 1 (WT-1); or optionally any of the tumor neoantigenic peptides disclosed in Int'l Pat. Pub. No. WO 2021/043804.

In some embodiments, the cancer is a myeloid cancer. In some embodiments, the cancer is a solid tumor. For example, the solid tumor is a cancer affecting an organ, optionally a cancer affecting colon, rectum, skin, endometrium, lung (including non-small cell lung carcinoma), uterus, bones (such as Osteosarcoma, Chondrosarcomas, Ewing's sarcoma, Fibrosarcomas, Giant cell tumors, Adamantinomas, and Chordomas), liver, kidney, esophagus, stomach, bladder, pancreas, cervix, brain (such as Meningiomas, Glioblastomas, Lower-Grade Astrocytomas, Oligodendrocytomas, Pituitary Tumors, Schwannomas, and Metastatic brain cancers), ovary, breast (such as triple negative breast cancer), head and neck region, testis, prostate or the thyroid gland.

In some embodiments, the chronic infectious disease is a chronic viral infection, optionally wherein the immune cell allows long term infection control and/or increased viral clearance. For example, the chronic viral infection is an infection with human immunodeficiency virus (HIV), Cytomegalovirus (CMV), Epstein-Barr virus (EBV), adenovirus, BK polyomavirus, hepatitis virus, such as hepatitis B, hepatitis C, hepatitis D, or hepatitis E.

In any of the embodiments, the SUV39H1 activity in the modified immune cell can be inactivated or inhibited. In some embodiments, the cell has been contacted with an exogenous SUV39H1 inhibitor, optionally a nucleic acid inhibitor of SUV39H1. In some embodiments, the exogenous SUV39H1 inhibitor is (a) a dominant negative inhibitor, or (b) an RNAi, shRNA, ribozyme or antisense oligonucleotide complementary to a fragment of the SUV39H1 gene, or (c) an epipolythiodioxopiperazine (ETP) class of SUV39H1 inhibitor.

In some embodiments, one or both SUV39H1 genes in the cell comprises one or more mutations that results in a deleted or non-functional SUV39H1 protein, or a SUV39H1 protein with reduced activity.

In some embodiments, the antigen-specific receptor is a modified TCR, or a chimeric antigen receptor (CAR). In some embodiments, cell comprises at least one antigen-specific receptor having an intracellular signaling domain wherein one or two immunoreceptor tyrosine-based activation motifs (ITAMs) are inactivated, optionally wherein the antigen-specific receptor comprises a single active ITAM domain. For example, the antigen-specific receptor is a chimeric antigen receptor (CAR) comprising: a) an extracellular antigen-binding domain that specifically binds an antigen, b) a transmembrane domain, c) optionally one or more costimulatory domains, and d) an intracellular signaling domain wherein one or two immunoreceptor tyrosine-based activation motifs (ITAMs) are inactivated; optionally wherein the antigen-specific receptor comprises a single active ITAM domain; and optionally wherein the intracellular domain comprises a modified CD3zeta intracellular signaling domain in which ITAM2 and ITAM3 have been inactivated.

In some embodiments, the antigen-specific receptor comprises an extracellular antigen-binding domain which is an scFv; or a single domain antibody; or an antibody heavy chain region (VH) and/or an antibody variable region (VL); or optionally a bispecific or trispecific antigen-binding domain. Optionally, the antigen-specific receptor comprises a transmembrane domain from CD28, CD8 or CD3-zeta, or a fragment thereof. Optionally, the antigen-specific receptor comprises one or more costimulatory domains selected from the group consisting of: 4-1BB (CD137), CD28, CD27, ICOS, OX40 (CD134) and DAP10; DAP12, 2B4, CD40, FCER1G and/or GITR (AITR), or an active fragment thereof.

In some embodiments, the antigen-specific receptor comprises: a) an extracellular antigen-binding domain that specifically binds an antigen, optionally comprising an antibody heavy chain variable region and/or an antibody light chain variable region, and is optionally bispecific or trispecific; b) a transmembrane domain, optionally comprising a fragment of transmembrane domain of alpha, beta or zeta chain of the T-cell receptor, CD28, CD3 epsilon, CD45, CD4, CD5, CD8, CD9, CD16, CD22, CD33, CD37, CD64, CD80, CD86, CD34, CD137, or CD154, NKG2D, OX40, ICOS, 2B4, DAP10, DAP12, CD40; c) optionally one or more costimulatory domains from 4-1BB, CD28, ICOS, OX40, DAP10 or DAP12, 2B4, CD40, FCER1G, or an active fragment thereof; and d) an intracellular signaling domain comprising an intracellular signaling domain from CD3zeta, FcR gamma, FcR beta, CD3 gamma, CD3 delta, CD3 epsilon, CDS, CD22, CD79a, CD79b, or CD66d, 2B4, or an active fragment thereof.

In some embodiments, the antigen-specific receptor is a chimeric antigen receptor (CAR) comprising: a) an extracellular antigen-binding domain, optionally an scFv, b) a transmembrane domain, optionally from CD28, CD8 or CD3-zeta, c) one or more co-stimulatory domains, optionally from 4-1 BB, CD28, ICOS, OX40 or DAP10, and d) an intracellular signaling domain from CD3zeta, optionally in which ITAM2 and ITAM3 have been inactivated.

In some embodiments, the antigen-specific receptor is a modified TCR that comprises a heterologous extracellular antigen-binding domain that specifically binds an antigen, optionally comprising an antibody heavy chain variable region and/or an antibody light chain variable region, and is optionally bispecific or trispecific. Optionally, the antigen-specific receptor is a modified TCR that comprises a fragment of an alpha, beta, gamma or epsilon chain and a heterologous extracellular antigen-binding domain that specifically binds an antigen, optionally comprising one or more single domain antibody, or an antibody heavy chain variable region and/or an antibody light chain variable region, optionally an scFv, and is optionally bispecific or trispecific. In some embodiments, modified TCR can be named recombinant HLA-independent (or non-HLA restricted) T cell receptors (referred to as"HI-TCRs") that bind to an antigen of interest in an HLA-independent manner. HLA independent (modified) TCRs are notably described in International Application No. WO 2019/157454. Such HI-TCRs comprise an antigen binding chain that comprises: (a) an antigen-binding domain that binds to an antigen in an HLA-independent manner, for example, an antigen-binding fragment of an immunoglobulin variable region; and (b) a constant domain that is capable of associating with (and consequently activating) a CD3ζ polypeptide. Because typically TCRs bind antigen in a HLA-dependent manner, the antigen-binding domain that binds in an HLA-independent manner must be heterologous.

The antigen-binding domain or fragment thereof comprises: a single domain antibody (VHH), or a heavy chain variable region (VH) of an antibody and/or a light chain variable region (VL) of an antibody. The constant domain of the TCR is, for example, a native or modified TRAC polypeptide, or a native or modified TRBC polypeptide. The constant domain of the TCR is, for example, a native TCR constant domain (alpha or beta) or fragment thereof. Unlike chimeric antigen receptors, which typically themselves comprise an intracellular signaling domain, the HI-TCR does not directly produce an activating signal; instead, the antigen-binding chain associates with and consequently activates a CD3ζ polypeptide. The immune cells comprising the recombinant TCR is highly sensitive and typically provide high activity when the targeted antigen is expressed at a low density (typically of less than about 10,000 molecules per cell) on the surface of a cell.

In some embodiments, the antigen-specific receptor is thus a modified TCR that comprises: a) a first antigen-binding chain comprising an antigen-binding fragment of a heavy chain variable region (VH) of an antibody; and b) a second antigen-binding chain comprising an antigen-binding fragment of a light chain variable region (VL) of an antibody; wherein the first and second antigen-binding chains each comprise a TRAC polypeptide or a TRBC polypeptide, optionally wherein at least one of the TRAC polypeptide and the TRBC polypeptide is endogenous, and optionally wherein one or both of the endogenous TRAC and TRBC polypeptides is inactivated.

In some embodiments, a heterologous nucleic acid sequence encoding the antigen-specific receptor or a portion thereof is inserted into the cell genome to express the antigen-specific receptor. In other embodiments, a heterologous nucleic acid sequence outside the cell genome expresses the antigen-specific receptor.

In some embodiments, insertion of the heterologous nucleic acid sequence encoding the antigen-specific receptor or a portion thereof inactivates expression of a native TCR alpha chain and/or a native TCR beta chain. In any of these embodiments, expression of the antigen-specific receptor may be under control of an endogenous promoter of a TCR, optionally an endogenous TRAC promoter.

In any of these embodiments, the immune cell is a T cell, a CD4+ T cell, a CD8+ T cell, a CD4+ and CD8+ T cell, a NK cell, a T regulatory cell, a TN cell, a memory stem T cell (TSCM), a TCM cell, a TEM cell, a monocyte, a dendritic cell, or a macrophage, or a progenitor thereof, optionally a T cell progenitor, a lymphoid progenitor, an NK cell progenitor, a myeloid progenitor, a pluripotent stem cell, an induced pluripotent stem cell (iPSC), a hematopoietic stem cell (HSC), an adipose derived stem cell (ADSC), or a pluripotent stem cell of myeloid or lymphoid lineage. Optionally, the immune cell is a T cell or NK cell, or progenitor thereof.

In any of these embodiments, the modified immune cell may further comprise a second engineered antigen-specific receptor, optionally a modified TCR or CAR, that specifically binds to a second antigen. For example, the modified immune cell comprises a first CAR that binds the antigen and a second CAR that binds a second antigen. As another example, the modified immune cell comprises a CAR that binds the antigen and modified TCR that binds a second antigen. As another example, the modified immune cell comprises a first modified TCR that binds the antigen and a second modified TCR that binds a second antigen. In some embodiments, the modified immune cell comprises three or more engineered antigen-specific receptors.

In any of these embodiments, the modified immune cell may further comprise a heterologous co-stimulatory receptor. In some embodiments, the co-stimulatory receptor comprises (a) an extracellular domain of a co-stimulatory ligand, optionally from CD80, (b) a transmembrane domain, optionally from CD80, and (c) an intracellular domain of a co-stimulatory molecule, optionally CD28, 4-1BB, OX40, ICOS, DAP10, CD27, CD40, NKGD2, or CD2, preferably 4-1BB.

In some embodiments, the extracellular antigen-binding domain binds, or comprises a VH and/or VL from an antibody that binds, an antigen with a KD affinity of about 1 × 10⁻⁷ M or less, about 5 × 10⁻⁸ M or less, about 1 × 10⁻⁸ M or less, about 5 × 10⁻⁹ M or less, about 1 × 10⁻⁹ M or less, about 5 × 10⁻¹⁰ M or less, about 1 × 10⁻¹⁰ M or less, about 5 × 10⁻¹¹ M or less, about 1 × 10⁻¹¹ M or less, about 5 × 10⁻¹² M or less, or about 1 × 10⁻¹² M or less (lower numbers indicating greater binding affinity).

In some embodiments, the antigen has a low density on the cell surface, of less than about 10,000, or less than about 5,000, or less than about 2,000 molecules per cell.

In some embodiments, SUV39H1 expression in the cell is reduced or inhibited by at least about 50%, 60%, 70%, 75%, 80%, 85%, 90% or 95%. In some embodiments, endogenous TCR expression of the cell is reduced by at least about 75%, 80%, 85%, 90% or 95%.

The modified immune cell may be allogeneic or autologous. For example, an HLA-A locus is inactivated in the cell. In some embodiments, HLA class I expression is reduced by at least about 75%, 80%, 85%, 90% or 95%.

In another aspect, the disclosure provides a method of producing the foregoing modified immune cell(s) comprising (a) introducing into the cell (a) a SUV39H1 inhibitor, and (b) (i) a nucleic acid encoding a CAR having one active ITAM or (ii) a heterologous nucleic acid encoding a portion of a modified TCR.

In some embodiments, the method or use comprises administering a CAR T-cell at a dose of less than about 5 × 10⁷ cells, optionally about 10⁵ to about 10⁷ cells. In some embodiments, the method or use comprises administering a second therapeutic agent, optionally one or more cancer chemotherapeutic agents, cytotoxic agents, cancer vaccines, hormones, anti-angiogens, radiolabelled compounds, immunotherapy, surgery, cryotherapy, and/or radiotherapy, to the subject.

In some embodiments, the second therapeutic agent is an immune checkpoint modulator is administered to the patient. For example, the immune checkpoint modulator is an antibody that specifically binds to, or other inhibitor of, PD1, PDL1, CTLA4, LAG3, BTLA, OX2R, TIM-3, TIGIT, LAIR-1, PGE2 receptor, EP2/4 adenosine receptor, or A2AR, optionally an anti-PD 1 or anti-PDL 1 antibody.

### BRIEF DESCRIPTION OF THE DRAWINGS

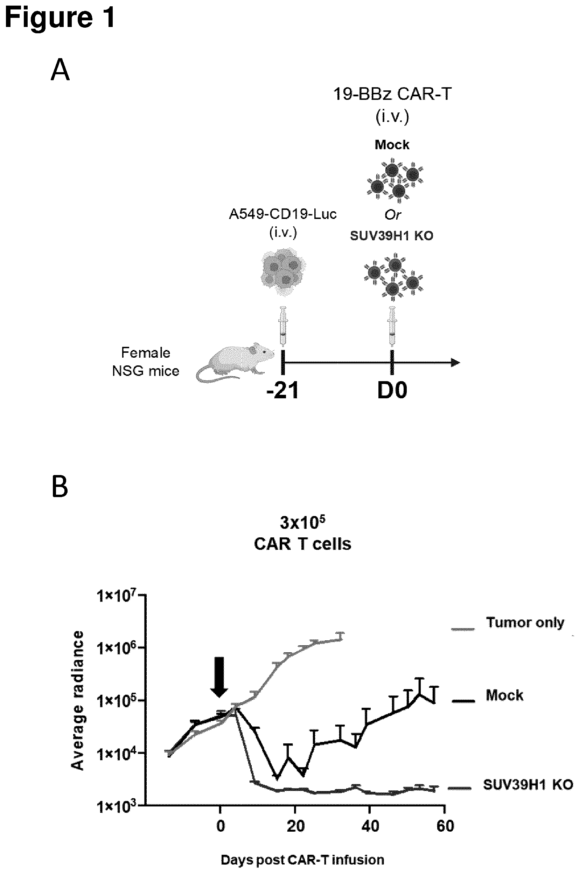

Figure 1A shows the schematic of the xenogeneic A549 lung adenocarcinoma model in NSG mice infused with 19-BBz CAR T cells (3×10⁵), either Mock (wildtype SUV39H1) or SUV39H1 KO. Figure 1B shows tumor growth measured by bioluminescence in the days post 19-BBz CAR T cell infusion (black arrow). SUV39H1 KO CAR T cells demonstrate better antitumor response (lower line).

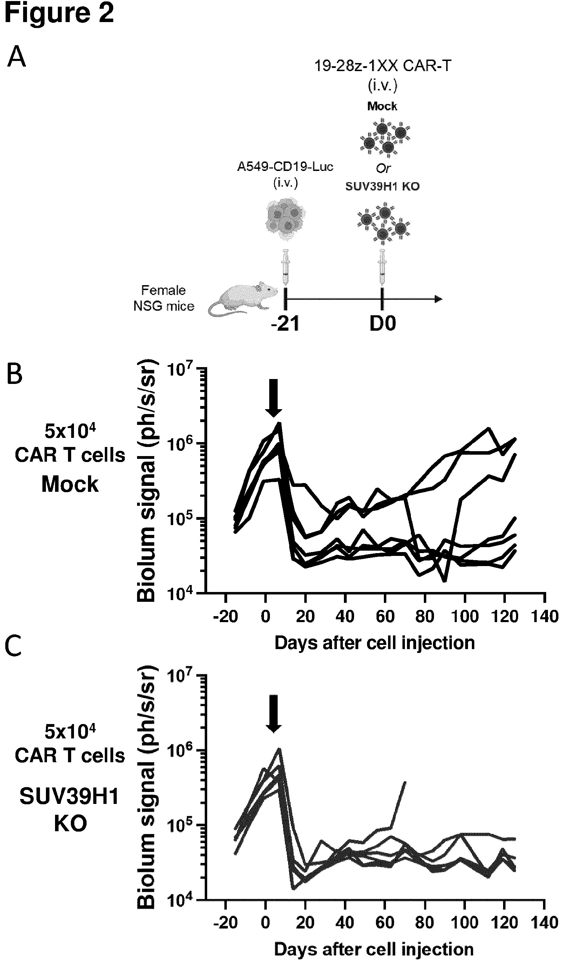

Figure 2A shows the schematic of the xenogeneic A549 lung adenocarcinoma model in NSG mice infused with 19-28z-1XX CAR T cells (5×10⁴), either Mock (wildtype SUV39H1) or SUV39H1 KO. Figures 2B and 2C show the bioluminescence measurements in NSG mice 69 days post Mock 19-28z-1XX CAR T cell infusion (Figure 2B) or SUV39H1 KO 19-28z-1XX CAR T cells (Figure 2C). There are 5 out of 7 mice treated with Mock CAR T cells that show tumor growth, in contrast to 2 out of 6 mice treated with SUV39H1 KO CAR T cells.

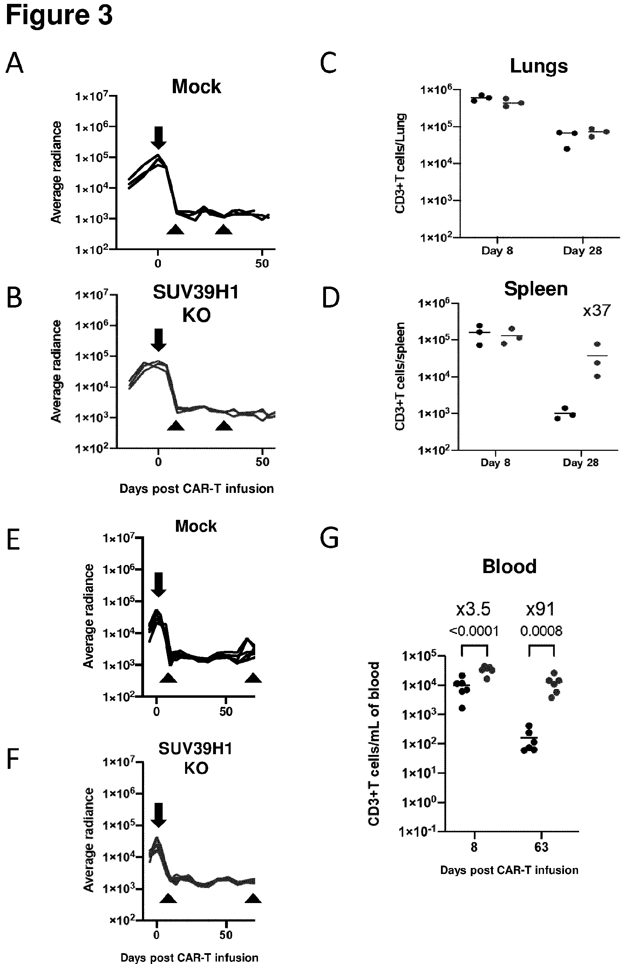

Figures 3A and 3B show tumor growth measured by bioluminescence in the days post 19-BBz Mock (Figure 3A) or SUV39H1 KO (Figure 3B) CAR T cell infusion (dose of 9×10⁵ cells, administered at time point indicated by black arrows). Black arrowheads indicate days when flow cytometry analysis was performed on lungs and spleens. Figures 3C and 3D show CAR T cell numbers in the lungs and spleens, respectively, of tumor bearing mice at two different time points (Day 8 and 28 post CAR T cell infusion). SUV39H1 KO CAR T cells are more abundant in the spleen after tumor eradication (Day 28). Figures 3E and 3F show tumor growth measured by bioluminescence in the days post 19-BBz Mock (Figure 3E) or SUV39H1 KO (Figure 3F) CAR T cell infusion (dose of 7.5×10⁵ cells, black arrows). Black arrowheads indicate days when flow cytometry analysis was performed on blood samples. Figure 3G shows CAR T cell numbers in the blood of tumor bearing mice at two different time points (Day 8 and 63 post CAR T cell infusion). SUV39H1 KO CAR T cells are more abundant in the blood at early time point (Day 8, initial expansion) and particularly at later time points (Day 28, persistence after tumor eradication).

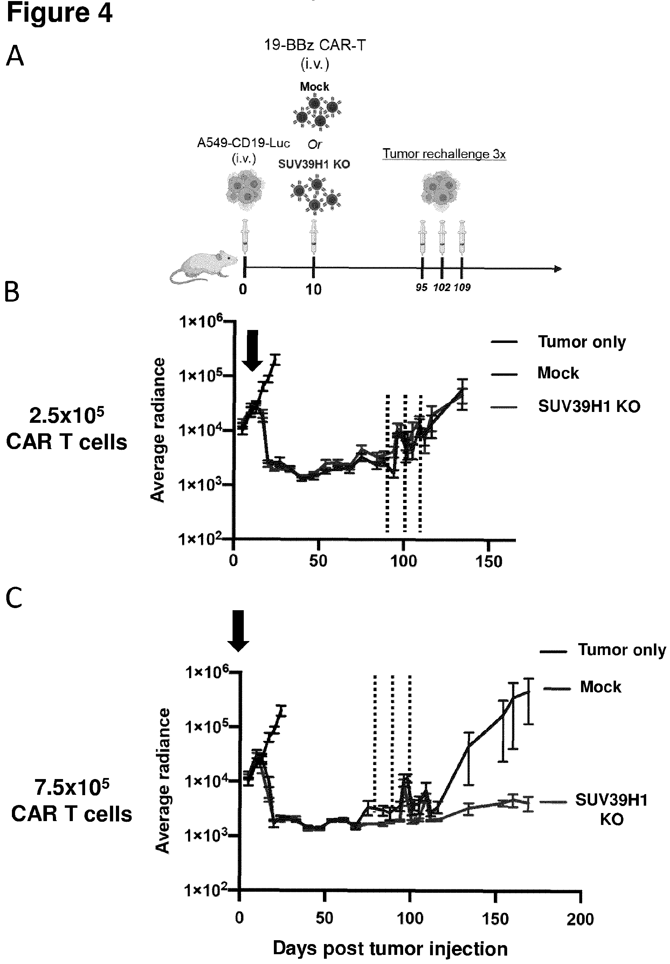

Figure 4A shows the schematic of the xenogeneic A549 lung adenocarcinoma rechallenge model in NSG mice infused with 19-BBz CAR T cells, either mock or SUV39H1 KO. Figure 4B shows tumor growth measured by bioluminescence in the days post infusion of 2.5×10⁵ 19-BBz CAR-T cells (administered at time point indicated by black arrow). At this dose, neither Mock nor SUV39H1 KO cells were able to control tumor rechallenges. Figure 4C shows tumor growth measured by bioluminescence in the days post infusion of 7.5×10⁵ 19-BBz CAR-T cells (black arrow). In both Figures 4B and 4C, vertical lines indicate dates of additional tumor cell injections (2×10⁶ A549-CD19 cells each). SUV39H1 KO CAR T cells demonstrate complete control of tumor rechallenges (lower line), while all Mock treated mice have relapses.

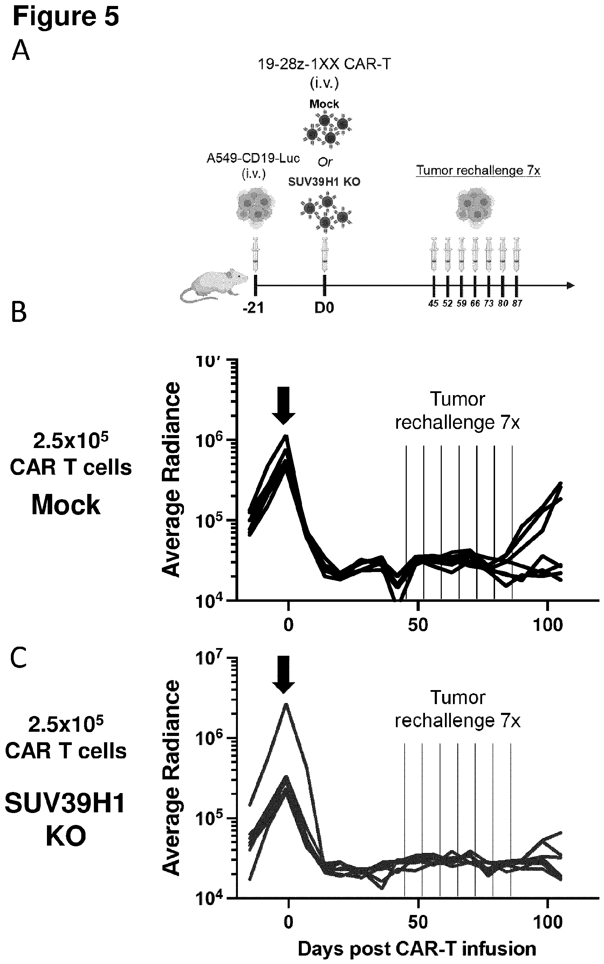

Figure 5A shows the schematic of the xenogeneic A549 lung adenocarcinoma rechallenge model in NSG mice infused with 19-28z-1XX CAR T cells, either mock or SUV39H1 KO. Figure 5B shows tumor growth measured by bioluminescence in the days post infusion of 2.5×10⁵ Mock (wild type SUV39H1) 19-28z-1XX CAR-T cells (black arrows). Figure 5C shows tumor growth measured by bioluminescence in the days post infusion of 2.5×10⁵ SUV39H1 KO 19-28z-1XX CAR-T cells (black arrows). In both Figures 5B and 5C, vertical lines indicate dates of additional tumor cell injections (2×10⁶ A549-CD19 cells each). SUV39H1 KO CAR T cells demonstrate complete control of tumor rechallenges (no mice have relapses), while 3 out of 7 Mock treated mice have tumor relapses.

### DETAILED DESCRIPTION OF THE INVENTION

SUV39H1 is a H3K9-histone methyltransferase that plays a role in silencing memory and stem cell programs during the terminal differentiation of effector CD8+ T cells. Silencing of SUV39H1, in turn, has been shown to enhance long-term memory potential and to increase survival capacity.

Immune cells, particularly T-cells or NK cells, in which SUV39H1 activity has been inactivated or inhibited have been shown herein to have surprisingly good efficacy against difficult to treat cancers, such as solid tumors, or refractory, relapsed or resistant cancer. Cells in which SUV39H1 activity has been inactivated or inhibited, and which also comprise an engineered chimeric antigen receptor (CAR) that has a single active ITAM domain are even more surprisingly efficacious. For immune cells in which expression of SUV39H1 has been inactivated, doses which are ineffective with a conventional CAR surprisingly become therapeutically effective with a CAR having a single active ITAM domain. Thus, the combination of SUV39H1 inactivation and a CAR having a single active ITAM domain (e.g. in which ITAM2 and ITAM3 have been inactivated) results in therapeutic efficacy at relatively lower doses of the cells. The cell therapy is also expected to be surprisingly beneficial for treating chronic infectious diseases.

### Definitions

As used herein, the singular forms "a," "an," and "the" are intended to include the plural forms as well, unless the context clearly indicates otherwise.

Also as used herein, "and/or" refers to and encompasses any and all possible combinations of one or more of the associated listed items, as well as the lack of combinations when interpreted in the alternative ("or").

The term "about," as used herein when referring to a measurable value such as an amount of polypeptide, dose, time, temperature, enzymatic activity or other biological activity and the like, is meant to encompass variations of ±10%, ±5%, ±1%, ±0.5%, or even ±0.1% of the specified amount.

The term "antibody" herein is used in the broadest sense and includes polyclonal and monoclonal antibodies, including intact antibodies and functional (antigen-binding) antibody fragments, including fragment antigen binding (Fab) fragments, F(ab')2 fragments, Fab' fragments, Fv fragments, recombinant IgG (rIgG) fragments, variable heavy chain (VH) regions capable of specifically binding the antigen, single chain antibody fragments, including single chain variable fragments (scFv), and single domain antibodies (e.g., sdAb, sdFv, nanobody) fragments. The term encompasses recombinant and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgG1, IgG2, IgG3, IgG4, IgM, IgE, IgA, and IgD. In some embodiments the antibody comprises a heavy chain variable region and a light chain variable region.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; variable heavy chain (VH) regions, VHH antibodies, single-chain antibody molecules such as scFvs and single-domain VH single antibodies; and multispecific antibodies formed from antibody fragments. In particular embodiments, the antibodies are single-chain antibody fragments comprising a variable heavy chain region and/or a variable light chain region, such as scFvs.

"Inactivation" or "disruption" of a gene refers to a change in the sequence of genomic DNA that causes the gene's expression to be reduced or eliminated, or that cause a non-functional gene product to be expressed. Exemplary methods include gene silencing, knockdown, knockout, and/or gene disruption techniques, such as gene editing through, e.g., induction of breaks and/or homologous recombination. Exemplary of such gene disruptions are insertions, frameshift and missense mutations, deletions, knock-in, and knock-out of the gene or part of the gene, including deletions of the entire gene. Such disruptions can occur in the coding region, e.g., in one or more exons, resulting in the inability to produce a full-length product, functional product, or any product, such as by insertion of a stop codon. Such disruptions may also occur by disruptions in the promoter or enhancer or other region affecting activation of transcription, so as to prevent transcription of the gene. Gene disruptions include gene targeting, including targeted gene inactivation by homologous recombination. Inactivation of a gene can decrease the activity of the expressed product by at least 20%, 30%, 40%, 50%, 60%, 70%, 80%, 90%, 95% or 99% or more as compared to the activity or expression levels of wildtype which is not inactivated.

As used herein, "inhibition" of a gene product refers to a decrease of its activity and/or gene expression of at least 20%, 30%, 40%, 50%, 60%, 70%, 80%, 90%, 95% or 99% or more as compared to the activity or expression levels of wildtype which is not inhibited or repressed.

As used herein, "express" or "expression" means that a gene sequence is transcribed, and optionally, translated. If the gene expresses a noncoding RNA, expression will typically result in an RNA after transcription and, optionally, splicing. If the gene is a coding sequence, expression will typically result in production of a polypeptide after transcription and translation.

As used herein, "expression control sequence" refers to a nucleotide sequence that influences the transcription, RNA processing, RNA stability, or translation of the associated nucleotide sequence. Examples include, but are not limited to, promoters, enhancers, introns, translation leader sequences, polyadenylation signal sequences, transcription initiators and transcriptional and/or translational termination region (i.e., termination region).

As used herein, "fragment" means a portion of a referenced sequence (polynucleotide or polypeptide) that has a length at least 60%, 65%, 70%, 75%, 80%, 85%, 90%, 95%, 96%, 97%, 98%, or 99% of the full-length sequence.

As used herein, "heterologous" refers to a polynucleotide or polypeptide that comprises sequences that are not found in the same relationship to each other in nature. For example, the heterologous sequence either originates from another species, or is from the same species or organism but is modified from either its original form or the form primarily expressed in the cell. Thus, a heterologous polynucleotide includes a nucleotide sequence derived from and inserted into the same natural, original cell type, but which is present in a non-natural state, e.g., a different copy number, and/or under the control of different regulatory sequences than that found in nature and/or located in a different position (adjacent to a different nucleotide sequence) than where it was originally located.

As used herein, "nucleic acid," "nucleotide sequence," and "oligonucleotide" or "polynucleotide" are used interchangeably and encompass both RNA and DNA, including cDNA, genomic DNA, mRNA, synthetic (e.g., chemically synthesized) DNA or RNA and chimeras of RNA and DNA. The term polynucleotide, nucleotide sequence, or nucleic acid refers to a chain of nucleotides without regard to length of the chain. The nucleic acid can be double-stranded or single-stranded. Where single-stranded, the nucleic acid can be a sense strand or an antisense strand. The nucleic acid can be synthesized using oligonucleotide analogs or derivatives (e.g., inosine or phosphorothioate nucleotides). Such oligonucleotides can be used, for example, to prepare nucleic acids that have altered base-pairing abilities or increased resistance to nucleases. The present disclosure further provides nucleic acid inhibitors that are complementary to a nucleic acid, nucleotide sequence, or polynucleotide described herein. Modified bases (modified nucleobases), such as inosine, 5-methylcytosine, 6-methyladenine, hypoxanthine and others can also be used for antisense, dsRNA, and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, or the 2'-hydroxy in the ribose sugar group of the RNA can also be made.

As used herein, "operably linked" means that an element, such as an expression control sequence, is configured so as to perform its usual function upon a nucleotide sequence of interest. For example, a promoter operably linked to a nucleotide sequence of interest is capable of effecting expression of the nucleotide sequence of interest. The expression control sequences need not be contiguous with the nucleotide sequence of interest, so long as they function to direct the expression thereof.

As used herein, the expression "percentage of identity" between two sequences, means the percentage of identical bases or amino acids between the two sequences to be compared, obtained with the best alignment of said sequences, this percentage being purely statistical and the differences between these two sequences being randomly spread over the two sequences. A base is considered complementary if it hybridizes under normal conditions. For example, a modified nucleobase can be aligned in a manner like the base whose hybridization pattern it mimics. As used herein, "best alignment" or "optimal alignment", means the alignment for which the determined percentage of identity (see above) is the highest. Sequence comparison between two nucleic acid sequences (also referenced herein as nucleotide sequence or nucleobase sequence) is usually realized by comparing these sequences that have been previously aligned according to the best alignment. This comparison is realized on segments of comparison in order to identify and compared the local regions of similarity. The best sequence alignment to perform comparison can be realized, besides manually, by using the global homology algorithm developed by SMITH and WATERMAN (Ad. App. Math., vol.2, p:482, 1981 ), by using the local homology algorithm developed by NEEDLEMAN and WUNSCH (J. Mol. Biol, vol.48, p:443, 1970), by using the method of similarities developed by PEARSON and LIPMAN (Proc. Natl. Acad. Sci. USA, vol.85, p:2444, 1988), by using computer software using such algorithms (GAP, BESTFIT, BLAST P, BLAST N, FASTA, TFASTA in the Wisconsin Genetics software Package, Genetics Computer Group, 575 Science Dr., Madison, WI, USA), by using the MUSCLE multiple alignment algorithms (Edgar, Robert C, Nucleic Acids Research, vol. 32, p: 1792, 2004). To get the best local alignment, one can preferably use BLAST software. The identity percentage between two sequences is determined by comparing these two sequences optimally aligned, the sequences being able to comprise additions or deletions with respect to the reference sequence in order to get the optimal alignment between these two sequences. The percentage of identity is calculated by determining the number of identical positions between these two sequences and dividing this number by the total number of compared positions, and by multiplying the result obtained by 100 to get the percentage of identity between these two sequences.

As used herein, "treatment", or "treating" involves application of cells of the disclosure or a composition comprising the cells to a patient in need thereof with the purpose to cure, heal, alleviate, relieve, alter, remedy, ameliorate, improve or affect the disease such as cancer, or any symptom of the disease (e.g., cancer or infectious disease). In particular, the terms "treat" or treatment" refers to reducing or alleviating at least one adverse clinical symptom associated with the disease. With reference to cancer treatment, the term "treat" or "treatment" also refers to slowing or reversing the progression of neoplastic uncontrolled cell multiplication, i.e. shrinking existing tumors and/or halting tumor growth. The term "treat" or "treatment" also refers to inducing apoptosis in cancer or tumor cells in the subject.

As used herein, "variant" means a sequence (polynucleotide or polypeptide) that has mutations (deletion, substitution or insertion) that is at least 60%, 65%, 70%, 75%, 80%, 85%, 90%, 95%, 96%, 97%, 98%, or 99% identical to a referenced sequence over its full length or over a region of at least about 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 200, 300, 400, 500, 600, 700, 800, 900, 1000, or 1100 nucleotides or amino acids. With respect to a polynucleotide sequence, variant also encompasses a polynucleotide that hybridizes under stringent conditions to the referenced sequence or complement thereof.

As used herein, a "vector" is any nucleic acid molecule for the transfer into or expression of a nucleic acid in a cell. The term "vector" includes both viral and nonviral (e.g., plasmid) nucleic acid molecules for introducing a nucleic acid into a cell *in vitro, ex vivo,* and/or *in vivo.* Vectors may include expression control sequences, restriction sites, and/or selectable markers. A "recombinant" vector refers to a vector that comprises one or more heterologous nucleotide sequences.

### Inhibitors of SUV39H1 expression or activity.

Human SUV39H1 sequence is provided in UniProt Accession No. O43463. The gene locus of SUV39H1 is located on the X chromosome (position p11.23, 48695554-48709016, in the GRCh38.p13 assembly). In some embodiments herein, SUV39H1 gene expression is reduced or eliminated by inactivation or disruption of one or both SUV39H1 genes. In other embodiments herein, SUV39H1 activity, as opposed to expression, is repressed. Known methods for gene repression include gene silencing, knockdown, knockout, and/or gene disruption techniques, such as gene editing.

SUV39H1 inhibitors can be gene editing agents; small molecule inhibitors; dominant negative inhibitors; antibody derivatives such as intrabodies, nanobodies or affibodies that typically block or inhibit Suv39h1 expression or activity; aptamers that typically block or inhibit Suv39h1 expression or activity; nucleic acid molecules that block or inhibit transcription or translation, such as antisense oligonucleotides complementary to Suv39h1; RNA interfering agents (such as a small interfering RNA (siRNA), small hairpin RNA (shRNA), microRNA (miRNA), or a piwiRNA (piRNA); ribozymes and combinations thereof.

Examples of inhibitors include gene editing techniques which result in targeted gene inactivation or disruption, e.g., by induction of breaks and/or homologous recombination. Gene editing agents include exogenous nuclease systems, or exogenous nucleic acid encoding the nuclease system. Examples include CRISPR-Cas systems comprising a) an engineered, non-naturally occurring Clustered Regularly Interspaced Short Palindromic Repeats (CRISPR) guide RNA that hybridizes with Suv39h1 genomic nucleic acid sequence and/or b) a nucleotide sequence encoding a CRISPR protein (e.g., a Type-II Cas9 protein or a Cpf1/Cas12 protein). The gene editing agent may also be a Zinc finger protein (ZFN) or a TAL protein.

Dominant negative inhibitors include mutant proteins without catalytic activity. Examples include SUV39H1 protein carrying an inactivating H324K point mutation within the HMT domain; or SUV39H1ΔSET, a deletion construct that lacks the catalytic domain. See Carbone et al. (2006), Mol. Cell. Bio., 26(4), 1288-1296.

Yet other inhibitors of SUV39H1 activity include small molecule inhibitors, for example, in the epipolythiodioxopiperazine (ETP) class.

The term "small organic molecule" refers to a molecule of a size comparable to those organic molecules generally used in pharmaceuticals. The term excludes biological macro molecules (e. g., proteins, nucleic acids, etc.). Preferred small organic molecules range in size up to about 5000 Da, more preferably up to 2000 Da, and most preferably up to about 1000 Da.

In one embodiment, the inhibitor of H3K9 -histone methyltransferase SUV39H1 is chaetocin (CAS 28097-03-2) as described by Greiner D, Bonaldi T, Eskeland R, Roemer E, Imhof A. "Identification of a specific inhibitor of the histone methyltransferase SU(VAR)3-9". Nat Chem Biol. 2005 Aug;1(3): 143-5.; Weber, H. P., et al, "The molecular structure and absolute configuration of chaetocin", Acta Cryst, B28, 2945-2951 (1972) ; Udagawa, S., et al, "The production of chaetoglobosins, sterigmatocystin, O-methylsterigmatocystin, and chaetocin by Chaetomium spp. and related fungi", Can. J. microbiol, 25, 170-177 (1979); and Gardiner, D. M., et al, "The epipolythiodioxopiperazine (ETP) class of fungal toxins: distribution, mode of action, functions and biosynthesis", Microbiol, 151, 1021-1032 (2005). For example, chaetocin is commercially available from Sigma Aldrich.

An inhibitor of SUV39H1 can also be ETP69 (Rac-(3S,6S,7S,8aS)-6-(benzo[d][1,3]dioxol-5-yl)-2,3,7-trimethyl-1,4-dioxohexahydro-6H-3,8a-epidithiopyrrolo[1,2-a]pyrazine-7-carbonitrile), a racemic analog of the epidithiodiketopiperazine alkaloid chaetocin A (see WO2014066435 but see also Baumann M, Dieskau AP, Loertscher BM, et al. Tricyclic Analogues of Epidithiodioxopiperazine Alkaloids with Promising In Vitro and In Vivo Antitumor Activity. Chemical science (Royal Society of Chemistry : 2010). 2015;6:4451-4457, and Snigdha S, Prieto GA, Petrosyan A, et al. H3K9me3 Inhibition Improves Memory, Promotes Spine Formation, and Increases BDNF Levels in the Aged Hippocampus. The Journal of Neuroscience. 2016;36(12):3611-3622).

The inhibiting activity of a compound may be determined using various methods as described in Greiner D. Et al. Nat Chem Biol. 2005 Aug;l(3): 143-5 or Eskeland, R. et al. Biochemistry 43, 3740-3749 (2004).

Inhibitors include intrabodies, which are antibodies that bind intracellularly to their antigen after being produced in the same cell. For a review, for example, see Marschall et al., MAbs. 2015;7(6):1010-35. Another intrabody format particularly suitable for cytoplasmic expression are single domain antibodies (also called nanobodies) derived from camels or consisting of one human VH domain or human VL domain. These single domain antibodies often have advantageous properties, e.g., high stability; good solubility; ease of library cloning and selection; high expression yield in E.coli and yeast.

Inhibitors include aptamers that inhibit or block SUV39H1 expression or activity. Aptamers are oligonucleotide (DNA or RNA) or oligopeptide sequences with the capacity to recognize virtually any class of target molecules with high affinity and specificity. Oligonucleotide aptamers may be isolated through Systematic Evolution of Ligands by Exponential enrichment (SELEX) of a random sequence library, as described in Tuerk C. and Gold L., 1990 or as reviewed in Jayasena S.D., 1999. Peptide aptamers may consist of a conformationally constrained antibody variable region displayed by a platform protein, such as *E. coli* Thioredoxin A that are selected from combinatorial libraries by two hybrid methods (Colas P, Cohen B, Jessen T, Grishina I, McCoy J, Brent R. "Genetic selection of peptide aptamers that recognize and inhibit cyclindependent kinase 2". Nature. 1996 Apr 11;380(6574):548-50).

Inhibitors include affibody molecules. Affibody are small proteins engineered to bind to a large number of target proteins or peptides with high affinity, imitating monoclonal antibodies, and are therefore a member of the family of antibody mimetics (see for review Löfblom J, Feldwisch J, Tolmachev V, Carlsson J, Ståhl S, Frejd FY. Affibody molecules: engineered proteins for therapeutic, diagnostic and biotechnological applications. FEBS Lett. 2010 Jun 18;584(12):2670-80). Affibody molecules are based on an engineered variant (the Z domain) of the B-domain in the immunoglobulin-binding regions of staphylococcal protein A, with specific binding for theoretically any given target.

Inhibitors include "nucleic acid inhibitors", which utilize hybridization or complementary to target gene repression. Examples include antisense oligonucleotides, RNAi, siRNA, shRNA, and/or ribozymes, all of which are well known in the art. For example, the nucleic acid inhibitors can be fully complementary to SUV39H1 mRNA over a stretch of about 12, 15, or 20 contiguous bases; or the inhibitors can be at least 90% complementary, or at least 80% complementary, or at least 70% complementary over the 12, 15 or 20 contiguous bases.

Typically, after transfection of siRNAs or expression of shRNAs, RNAi triggers the degradation of target RNA molecules through direct complementarity, mediated by the RNA-induced silencing complex. An alternative to RNAi for the degradation of IncRNAs are ASOs. ASOs are 15-20-nucleotide single-stranded DNA oligomers that are typically chemically modified to increase the efficacy of knockdown and decrease in vivo toxicity. In particular, the 2'-MOE and LNA gapmer modifications have been shown to increase affinity toward target RNA transcripts and endow resistance to nucleases, allowing these modified ASOs to have half-lives between days to several weeks in vivo. ASOs hybridize with target RNA transcripts through complementarity and induce RNaseH-mediated degradation of the target transcripts.

RNA chemical modifications include one or more of a modified backbone linkage, a modified sugar moiety, a modified phosphate moiety, a modified nucleobase, or a chemically conjugated moiety. Such chemical modifications can be present throughout the polynucleotide, or in alternating patterns. Chemical modifications can be present at the 5' end or 3' end or both, e.g. the 5-10 bases at the 5' and/or 3' end comprise one or more of a modified backbone linkage, a modified sugar moiety, a modified phosphate moiety, a modified nucleobase, or a chemically conjugated moiety.

Such nucleic acid inhibitors can be delivered directly, as heterologous RNA or as heterologous polynucleotides, e.g., chemically modified polynucleotides that have been modified to increase their serum half-life and/or affinity. Alternatively, such nucleic acid inhibitors can be expressed ectopically from DNA or vectors. In yet another alternative, endogenous expression of such nucleic acid inhibitors can be upregulated, e.g., by inserting expression control sequences, such as constitutive, inducible, strong or tissue-specific promoters.

Inhibition of SUV39H1 in the cell can be achieved before or after injection in the targeted patient. In some embodiment, inhibition as described herein is performed in vivo after administration of the cell to the subject. In some embodiments, a SUV39H1 inhibitor as herein defined can be included in the composition containing the cell. SUV39H1 inhibitors may also be administered separately before, concomitantly of after administration of the cell(s) to the subject.

In some embodiments, inhibition of SUV39H1 according to the invention may be achieved with incubation of a cell with a composition containing at least one SUV39H1 inhibitor, or introduction of at least SUV39H1 inhibitor into the cell, or introduction of one or more nucleic acids encoding a SUV39H1 inhibitor into the cell, as previously described. The SUV39H1 inhibitor is included during the proliferation or expansion of the modified immune cells *in vitro.*

### Expression of proteins or RNA

Means and vectors for expressing polypeptides or polynucleotides such as RNA are well known in the art and commercially available. Known vectors include viral vectors and pseudotyped viral vectors, such as retrovirus (e.g., moloney murine leukemia virus, harvey murine sarcoma virus, murine mammary tumor virus, and rous sarcoma virus), lentivirus, adenovirus, adeno-associated virus (AAV), alphavirus, vaccinia virus, poxvirus, SV40-type viruses, polyoma viruses, Epstein-Barr viruses, herpes simplex virus, papilloma virus, polio virus, foamivirus, or Semliki Forest virus vectors; or transposase systems, such as Sleeping Beauty transposase vectors. Non-viral systems for delivery of naked plasmids to cells include lipofection, nucleofection, microinjection, biolistics, virosomes, lipids, cationic lipid complexes, liposomes, immunoliposomes, nanoparticle, gold particle, or polymer complex, poly-lysine conjugates, synthetic polyamino polymers, other agent-enhanced uptake of DNA, and artificial viral envelopes or virions.

For expressing short noncoding RNAs, such as shRNA, polymerase III promoters are commonly used. Examples include U6, HI, or 7SK promoters.

For expressing long RNAs, such as mRNAs or long noncoding RNAs, polymerase II promoters are commonly used. Examples include CMV, EF-1a, hPGK and RPBSA. CAG promoters have been used to overexpress IncRNA. Yin et al Cell Stem Cell. 2015 May 7;16(5):504-16. Inducible promoters that are driven by signals from activated T cells include nuclear factor of activated T cells (NFAT) promoter. Other promoters for T cell expression of RNA include CIFT chimeric promoter (containing portions of cytomegalovirus (CMV) enhancer, core interferon gamma (IFN-γ) promoter, and a T-lymphotropic virus long terminal repeat sequence (TLTR)), endogenous TRAC promoter or TRBC promoter. Inducible, constitutive, or tissue-specific promoters are contemplated.

In some embodiments, dsRNA such as RNAi is produced in the cell by a vector comprising an expression control sequence operatively linked to a nucleotide sequence that expresses (is a template for one or both strands of) the dsRNA. In further embodiments, a promoter can flank either end of the template nucleotide sequence, wherein the promoters drive expression of each individual DNA strand, thereby generating two complementary (or substantially complementary) RNAs that hybridize and form the dsRNA. In other embodiments, dsRNA is produced in the cell by a vector that expresses an shRNA that is processed to form an interfering dsRNA.

### Delivery of nucleic acids or polynucleotides, including vectors

Art-recognized techniques for introducing foreign nucleic acids (e.g., DNA and RNA) into a host cell, include calcium phosphate or calcium chloride co-precipitation, DEAE-dextran-mediated transfection, lipofection, electroporation, microinjection, DNA-loaded liposomes, lipofectamine-DNA complexes, cell sonication, gene bombardment using high velocity microprojectiles, biolistics, and viral-mediated transfection. Compositions comprising the nucleic acids may also comprise transfection facilitating agents, which include surface active agents, quinone analogs, vesicles such as squalene and squalene, hyaluronic acid, lipids, liposomes, lecithin liposomes, calcium ions, viral proteins, polyanions, polycations, including poly-L-glutamate, or nanoparticles, gold particles, or other known agents. Delivery vehicles include a liposome, lipid-containing complex, nanoparticle, gold particle, or polymer complex.

Lipid materials have been used to create lipid nanoparticles (LNPs) based on ionizable cationic lipids, which exhibit a cationic charge in the lowered pH of late endosomes to induce endosomal escape, because of the tertiary amines in their structure. These LNPs have been used, for example, to deliver RNA interference (RNAi) components, as well as genetic constructs or CRISPR-Cas systems. See, such as, Wilbie et al., Acc Chem Res.;52(6): 1555-1564, 2019. Wang et al., Proc Natl Acad Sci U S A.;113(11):2868-2873, 2016 describe use of biodegradable cationic LNPs. Chang et al., Acc. Chem. Res., 52, 665-675, 2019 describe use of ionizable lipid along with cholesterol, DSPC, and a PEGylated lipid to create LNPs.

Polymer based particles can be used for genetic construct delivery in a similar manner as lipids. Numerous materials have been used for delivery of nucleic acids. For example, cationic polymers such as polyethylenimine (PEI) can be complexed to nucleic acids and can induce endosomal uptake and release, similarly to cationic lipids. Dendrimeric structures of poly(amidoamine) (PAMAM) can also be used for transfection. These particles consist of a core from which the polymer branches. They exhibit cationic primary amines on their surface, which can complex to nucleic acids. Networks based on zinc to aid cross-linking of imidazole have been used as delivery methods, relying on the low pH of late endosomes which, upon uptake, results in cationic charges due to dissolution of the zeolitic imidazole frameworks (ZIF), after which the components are released into the cytosol. Colloidal gold nanoparticles have also been used. See Wilbie et al., supra.

### Production and chemical modification of nucleic acid inhibitors

In vitro transcribed, chemically synthesized, or partially chemically synthesized RNAs can be delivered. For example, an RNA may be in vitro transcribed and chemically linked to chemically synthesized RNA at the 5' end and/or 3' end. Direct injection or transfection of in vitro-transcribed IncRNAs has been performed to demonstrate IncRNA function. Ulitsky et al. (2011) Cell, 147(7): 1537-1550.

Chemical modifications to oligonucleotides (also referred to as polynucleotides) can improve their resistance to degradation, thereby increasing half-life, and/or increase affinity for complementary polynucleotides. Modified oligonucleotides (e.g., RNA oligonucleotides) comprise chemical modifications including one or more of a modified backbone linkage, a modified phosphate moiety, a modified sugar moiety, a modified nucleobase, or a chemically conjugated moiety, or any combinations thereof. One, two, three, four, five, 10, 15, 20 or more of the same type of modification, optionally in combination with 1, 2, 3, 4, 5, 10, 15, 20 or more of another type of modification, or patterns of modifications are contemplated. Patterns include alternating modifications throughout the oligonucleotide, such as 2'-fluoro and 2'-methoxy, or end modifications wherein, for example, 1, 2, 3, 4, 5, or more of the bases, sugars, or linkages at the 5' and/or 3' end of the oligonucleotide are modified.

Examples of modified backbone linkages include phosphorothioate, phosphothioate (PhTx) group or phosphonoacetate, thiophosphonoacetate, methylphosphonate, boranophosphate, or phosphorodithioate. Other internucleotide bridging modified phosphates may be used, such as methyl phosphonothioates, phosphoromorpholidates, phosphoropiperazidates and phosphoramidates. For example, every one or every other one of the internucleotide bridging phosphate residues can be modified as described.

Examples of modified sugar moieties include deoxyribose, or replacement of the 2' OH-group by another group. Although the majority of sugar analog alterations are localized to the 2' position, other sites are amenable to modification, including the 4' position. Example replacement groups include H, -OR, -R (wherein R can be, such as, alkyl, cycloalkyl, aryl, aralkyl, heteroaryl or sugar), lower alkyl moiety (e.g., C1-C4, linear or branched, saturated or unsaturated alkyl, such as methyl, ethyl, ethenyl, propyl, 1-propenyl, 2-propenyl, and isopropyl), halo, -F, -Br, -Cl or -I, -SH, -SR (wherein R can be, such as, alkyl, cycloalkyl, aryl, aralkyl, heteroaryl or sugar), -arabino, F-arabino, amino (wherein amino can be, such as, NH₂; alkylamino, dialkylamino, heterocyclyl, arylamino, diarylamino, heteroarylamino, diheteroarylamino, or amino acid); or cyano (-CN). Specific examples include a 2'-fluoro sugar, 2'-O-methyl sugar, 2'-O-methoxyethyl sugar, or a locked nucleic acid (LNA) nucleotide. Specific examples of modified 2'-sugar include 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aeo), 2'-O-methoxyethyl-5-methylcytidine (m5Ceo), and any combinations thereof. For example, every one or every other one of the nucleotides can be modified as described.

Examples of modified nucleobases include, but are not limited to, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomet-hyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

Polynucleotides can also be stabilized by complexing to lipids or liposomes. In some embodiments, the liposome comprises 1,2-dioleoyl-sn-glycero-3-phosphatidylcholine (DOPC). In certain embodiments, the lipid particle comprises cholesterol, 1,2-distearoyl-sn-glycero-3-phosphocholine (DSPC), PEG-cDMA or PEG-cDSA, and 1,2-dilinoleyloxy-3-(N,N-dimethyl)aminopropane (DLinDMA).

### Cells with modulated SUV39H1 expression

Cells according to the disclosure exhibit modulation, preferably inhibition, of SUV39H1 expression. The cells are typically mammalian cells, or cell lines, e.g., mouse, rat, pig, non-human primate, or preferably human. Such cells include cells derived from the blood, bone marrow, lymph, or lymphoid organs (notably the thymus) and are preferably cells of the immune system (i.e., immune cells), such as cells of the innate or adaptive immunity, e.g., myeloid or lymphoid cells, including monocytes, macrophages, dendritic cells, or lymphocytes, typically T cells and/or NK cells. Immune cells or progenitors thereof preferably also comprise one or more, or two or more, or three or more antigen-specific receptors (CAR and/or TCR) as described herein, and optionally comprise one or more co-stimulatory receptors. Among the antigen-specific receptors according to the disclosure are recombinant modified T cell receptors (TCRs) and components thereof, as well as functional non-TCR antigen-specific receptors, such as chimeric antigen receptors (CAR).

Cells according to the disclosure may also be immune cell progenitors, such as lymphoid progenitors and more preferably T cell progenitors. Examples of T-cell progenitors include pluripotent stem cells (PSC), induced pluripotent stem cells (iPSCs), hematopoietic stem cells (HSC), human embryonic stem cells (ESC), adipose-derived stem cells (ADSC), multipotent progenitor (MPP); lymphoid-primed multipotent progenitor (LMPP); common lymphoid progenitor (CLP); lymphoid progenitor (LP); thymus settling progenitor (TSP); or early thymic progenitor (ETP). Hematopoietic stem and progenitor cells can be obtained, for example, from cord blood, or from peripheral blood, e.g. peripheral blood- derived CD34+ cells after mobilization treatment with granulocyte-colony stimulating factor (G-CSF). T cell progenitors typically express a set of consensus markers including CD44, CD117, CD135, and/or Sca-1.

In some embodiments, the cells include one or more subsets of T cells or other cell types, such as whole T cell populations, CD4+ and/or CD8+ T cells, and subpopulations thereof, such as those defined by function, activation state, maturity, potential for differentiation, expansion, recirculation, localization, and/or persistence capacities, antigen-specificity, type of antigen-specific receptor, presence in a particular organ or compartment, marker or cytokine secretion profile, and/or degree of differentiation. In some embodiments, the cells include myeloid derived cells, such as dendritic cells, monocytes or macrophages.

Among the sub-types and subpopulations of T cells and/or of CD4+ and/or of CD8+ T cells are naive T (TN) cells, effector T cells (TEFF), memory T cells and sub-types thereof, such as stem cell memory T (TSCM), central memory T (TCM), effector memory T (TEM), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells. Specifically contemplated herein are TEFF cells with stem/memory properties and higher reconstitution capacity due to the inhibition of SUV39H1, as well as TN cells, TSCM, TCM, TEM cells and combinations thereof.

In some embodiments, one or more of the T cell populations is enriched for, or depleted of, cells that are positive for or express high levels of one or more particular markers, such as surface markers, or that are negative for or express relatively low levels of one or more markers. In some cases, such markers are those that are absent or expressed at relatively low levels on certain populations of T cells (such as non-memory cells) but are present or expressed at relatively higher levels on certain other populations of T cells (such as memory cells). In one embodiment, the cells (such as the CD8+ cells or the T cells, e.g., CD3+ cells) are enriched for (i.e., positively selected for) cells that are positive or expressing high surface levels of CD117, CD135, CD45RO, CCR7, CD28, CD27, CD44, CD127, and/or CD62L and/or depleted of (e.g., negatively selected for) cells that are positive for or express high surface levels of CD45RA. In some embodiments, cells are enriched for or depleted of cells positive or expressing high surface levels of CD122, CD95, CD25, CD27, and/or IL7-Ra (CD127). In some examples, CD8+ T cells are enriched for cells positive for CD45RO (or negative for CD45RA) and for CD62L. The subset of cells that are CCR7+, CD45RO+, CD27+, CD62L+ cells constitute a central memory cell subset.

For example, according to the disclosure, the cells can include a CD4+ T cell population and/or a CD8+ T cell sub-population, e.g., a sub-population enriched for central memory (TCM) cells. Alternatively, the cells can be other types of lymphocytes, including natural killer (NK) cells, mucosal associated invariant T (MAIT) cells, Innate Lymphoid Cells (ILCs) and B cells.

Cells include primary cells, isolated directly from a biological sample obtained from a subject, and optionally frozen. In some embodiments, the subject is in need of a cell therapy (adoptive cell therapy) and/or is the one who will receive the cell therapy. With reference to a subject to be treated with cell therapy, the cells may be allogeneic and/or autologous. In autologous immune cell therapy, immune cells are collected from the patient, modified as described herein, and returned to the patient. In allogeneic immune cell therapy, immune cells are collected from healthy donors, rather than the patient, modified as described herein, and administered to patients. Typically, these are HLA matched to reduce the likelihood of rejection by the host. The immune cells may also comprise modifications to reduce immunogenicity such as disruption or removal of HLA class I molecules, HLA-A locus, and/or Beta-2 microglobulin (B2M).

Universal "off the shelf product" immune cells typically comprise modifications designed to reduce graft vs. host disease, such disruption or deletion of endogenous TCR. Because a single gene encodes the alpha chain (TRAC) rather than the two genes encoding the beta chain (TRBC), the TRAC locus is a common target for removing or disrupting endogenous TCR expression.

The samples include tissue samples, from tissues or organ, or fluid samples, such as blood, plasma, serum, cerebrospinal fluid, or synovial fluid. Samples may be taken directly from the subject, or may result from one or more processing steps, such as separation, centrifugation, genetic engineering (for example transduction with viral vector), washing, and/or incubation. Blood or blood-derived samples may be derived from an apheresis or a leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, myeloid derived cells, and/or cells derived therefrom.

### Methods for producing cells

Provided herein are methods of producing the cells of the disclosure with modulated SUV39H1 expression. For cells with inhibited SUV39H1 expression, such methods include steps of, for example, (a) introducing into the cell an exogenous SUV39H1 inhibitor, or nucleic acid(s) encoding an exogenous SUV39H1 inhibitor, to disrupt or inactivate or inhibit SUV39H1 activity. In some embodiments, the SUV39H1 gene can be disrupted by introducing a gene editing agent to delete all or part of the gene, or to mutate the gene to produce a truncated or non-functional protein.

Such methods may further include steps of (b) introducing a nucleic acid encoding all or part of an antigen-specific receptor, e.g. introducing a nucleic acid encoding an antibody fragment, and optionally (c) introducing a nucleic acid encoding a chimeric co-stimulatory receptor.

### Antigen-specific receptors

The cells of the disclosure with modulated SUV39H1 expression include immune cells that express one or more, or two or more, or three or more antigen-specific receptors on their surface, and optionally one or more co-stimulatory receptors. Antigen-specific receptors include recombinant or modified T cell receptors (TCRs) and components thereof, and/or chimeric antigen receptors (CAR). For example, at least two CAR, at least two TCR, or at least one CAR with at least one TCR can be contemplated. The two or more antigen-specific receptors may bind the same or different antigen. In some embodiments, the two or more antigen-specific receptors have different signaling domains. In some embodiments, the cell comprises an antigen-specific receptor with an activating signaling domain and an antigen-specific receptor with an inhibitory signaling domain. Typically, such antigen-specific receptors bind the target antigen with a Kd binding affinity of about 10⁻⁶M or less, about 10⁻⁷M or less, about 10⁻⁸M or less, about 10⁻⁹M or less, about 10⁻¹⁰M or less, or about 10⁻¹¹M or less (lower numbers indicating greater binding affinity).

The cells thus may comprise one or more nucleic acids that encode one or more antigen-specific receptors, optionally operably linked to a heterologous regulatory control sequence. Typically, the nucleic acids are heterologous, (i.e., for example which are not ordinarily found in the cell being engineered and/or in the organism from which such cell is derived). In some embodiments, the nucleic acids are not naturally occurring, including chimeric combinations of nucleic acids encoding various domains from multiple different cell types. The nucleic acids and their regulatory control sequences are typically heterologous. For example, the nucleic acid encoding the antigen-specific receptor may be heterologous to the immune cell and operatively linked to an endogenous promoter of the T-cell receptor such that its expression is under control of the endogenous promoter. In some embodiments, the nucleic acid encoding a CAR is operatively linked to an endogenous TRAC promoter.

The immune cells, particularly if allogeneic, may be designed to reduce graft vs. host disease, such that the cells comprise inactivated (e.g. disrupted or deleted) endogenous TCR. Because a single gene encodes the alpha chain (TRAC) rather than the two genes encoding the beta chain, the TRAC locus is a typical target for reducing TCR receptor expression. Thus, the nucleic acid encoding the antigen-specific receptor (e.g. CAR or TCR) may be integrated into the TRAC locus at a location (for example in the 5' region of the first exon (SEQ ID NO: 3 of WO 2017/180989), that significantly reduces expression of a functional TCR alpha chain. See, e.g., Jantz et al., WO 2017/062451; Sadelain et al.,; Torikai et al., Blood, 119(2): 5697-705 (2012); Eyquem et al., Nature. 2017 Mar 2;543(7643): 113-117. Expression of the endogenous TCR alpha may be reduced by at least 50%, 60%, 70%, 75%, 80%, 85%, 90%, 95%, 98%, or 99%. In such embodiments, expression of the nucleic acid encoding the antigen-specific receptor is optionally under control of the endogenous TCR-alpha promoter.

### Chimeric Antigen Receptors (CARs)

In some embodiments, the engineered antigen-specific receptors comprise chimeric antigen receptors (CARs), including activating or stimulatory CARs, costimulatory CARs (see WO2014/055668), and/or inhibitory CARs (iCARs, see Fedorov et al., Sci. Transl. Medicine, 5(215) (December 2013)).

Chimeric antigen receptors (CARs), (also known as Chimeric immunoreceptors, Chimeric T cell receptors, Artificial T cell receptors) are engineered antigen-specific receptors, which graft an arbitrary specificity onto an immune effector cell (T cell). Typically, these receptors are used to graft the specificity of a monoclonal antibody onto a T cell, with transfer of their coding sequence facilitated by retroviral vectors.

CARs generally include an extracellular antigen (or ligand) binding domain linked to one or more intracellular signaling components, in some aspects via linkers and/or transmembrane domain(s). Such molecules typically mimic or approximate a signal through a natural antigen receptor, a signal through such a receptor in combination with a costimulatory receptor, and/or a signal through a costimulatory receptor alone.

The CAR may include (a) an extracellular antigen-binding domain, (b) a transmembrane domain, (c) optionally a co-stimulatory domain, and (d) an intracellular signaling domain.

In some embodiments, the CAR is constructed with a specificity for a particular antigen (or marker or ligand), such as an antigen expressed in a particular cell type to be targeted by adoptive cell therapy, such as a cancer marker. The CAR typically includes in its extracellular portion one or more antigen binding molecules, such as one or more antigen-binding fragment, domain, or portion of an antibody, typically one or more antibody variable domains. For example, the extracellular antigen-binding domain may comprise a light chain variable domain or fragment thereof and/or a heavy chain variable domain or fragment thereof, typically as an scFv. In some embodiments, the CAR comprises an antibody heavy chain variable domain or fragment thereof that specifically binds the antigen.

The moieties used to bind to antigen include three general categories, either single-chain antibody fragments (scFvs) derived from antibodies, Fab's selected from libraries, or natural ligands that engage their cognate receptor (for the first-generation CARs). Successful examples in each of these categories are notably reported in Sadelain M, Brentjens R, Riviere I. The basic principles of chimeric antigen receptor (CAR) design. Cancer discovery. 2013; 3(4):388-398 (see notably table 1) and are included in the present disclosure.

Antibodies include chimeric, humanized or human antibodies, and can be further affinity matured and selected as described above. Chimeric or humanized scFvs derived from rodent immunoglobulins (e.g. mice, rat) are commonly used, as they are easily derived from well-characterized monoclonal antibodies. Humanized antibodies contain rodent-sequence derived CDR regions. Typically the rodent CDRs are engrafted into a human framework, and some of the human framework residues may be back-mutated to the original rodent framework residue to preserve affinity, and/or one or a few of the CDR residues may be mutated to increase affinity. Fully human antibodies have no murine sequence and are typically produced via phage display technologies of human antibody libraries, or immunization of transgenic mice whose native immunoglobin loci have been replaced with segments of human immunoglobulin loci. Variants of the antibodies can be produced that have one or more amino acid substitutions, insertions, or deletions in the native amino acid sequence, wherein the antibody retains or substantially retains its specific binding function. Conservative substitutions of amino acids are well known and described above. Further variants may also be produced that have improved affinity for the antigen.

In some embodiments, the modified TCR or CAR contains a fragment of an antibody or an antigen-binding fragment (e.g. single domain antibody, scFv, or variable heavy (VH) region and/or variable light (VL) region or 1, 2, or 3 CDRs of such VH and/or VL) that specifically recognizes an intracellular antigen, such as a tumor-associated antigen, presented on the cell surface as a MHC-peptide complex. Generally, a CAR containing an antibody or antigen-binding fragment that exhibits TCR-like specificity directed against peptide-MHC complexes also may be referred to as a TCR-like CAR.

The transmembrane domain in some embodiments is derived either from a natural or from a synthetic source. The transmembrane domain may be derived from the same receptor as the intracellular signaling domain, or a different receptor. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD 134, CD137, CD154, ICOS or a GITR, or NKG2D, OX40, 2B4, DAP10, DAP12, or CD40. For T cells, CD8, CD28, CD3 epsilon may be preferred. For NK cells, NKG2D, DAP10, DAP12 may be preferred. In some embodiments, the transmembrane domain is derived from CD28, CD8 or CD3-zeta. In some instances, the transmembrane domain is selected or modified by amino acid substitution to avoid binding of such domains to the transmembrane domains of the same or different surface membrane proteins to minimize interactions with other members of the receptor complex.

In some embodiments, a short oligo- or polypeptide linker, for example, a linker of between 2 and 10 amino acids in length, is present and forms a linkage between the transmembrane domain and the cytoplasmic signaling domain of the CAR.

The CAR generally includes at least one intracellular signaling component or components. First generation CARs typically had the intracellular domain from the CD3-zeta-chain, which is the primary transmitter of signals from endogenous TCRs. Second generation CARs typically further comprise intracellular signaling domains from various costimulatory protein receptors to the cytoplasmic tail of the CAR to provide additional signals to the T cell. Costimulatory domains include domains derived from human CD28, 4-1BB (CD137), ICOS, CD27, OX 40 (CD134), DAP10, DAP12, 2B4, CD40, FCER1G or GITR (AITR). For T cells, CD28, CD27, 4-1BB (CD137), ICOS may be preferred. For NK cells, DAP10, DAP12, 2B4 may be preferred. Combinations of two co-stimulatory domains are contemplated, e.g. CD28 and 4-1BB, or CD28 and OX40. Third generation CARs combine multiple signaling domains, such as CD3zeta-CD28-4-1BB or CD3zeta-CD28-OX40, to augment potency.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co- stimulatory signal (secondary cytoplasmic signaling sequences). In some aspects, the CAR includes one or both of such signaling components.

In some aspects, the CAR includes a primary cytoplasmic signaling sequence that regulates primary activation of the TCR complex either in a stimulatory way, or in an inhibitory way. Primary cytoplasmic signaling sequences that act in a stimulatory manner may contain signaling motifs which are known as immunoreceptor tyrosine -based activation motifs or ITAMs. Examples of ITAM containing primary cytoplasmic signaling sequences include those derived from TCR zeta, FcR gamma, FcR beta, CD3 gamma, CD3 delta, CD3 epsilon, CDS, CD22, CD79a, CD79b, and CD66d. In some embodiments, cytoplasmic signaling molecule(s) in the CAR contain(s) a cytoplasmic signaling domain, portion thereof, or sequence derived from CD3 zeta. The CAR can also include a signaling domain and/or transmembrane portion of a costimulatory receptor, such as CD28, 4-1BB (CD137), ICOS, CD27, OX 40 (CD134), DAP10, DAP12, 2B4, CD40, FCER1G or GITR (AITR). In some aspects, the same CAR includes both the activating and costimulatory components; alternatively, the activating domain is provided by one CAR whereas the costimulatory component is provided by another CAR recognizing another antigen.

The intracellular signaling domain can be from an intracellular component of the TCR complex, such as a TCR CD3 chain that mediates T-cell activation and cytotoxicity, e.g., the CD3 zeta chain. Alternative intracellular signaling domains include FcεRIγ. The intracellular signaling domain may comprise a modified CD3 zeta polypeptide lacking one or two of its three immunoreceptor tyrosine-based activation motifs (ITAMs), wherein the ITAMs are ITAM1, ITAM2 and ITAM3 (numbered from the N- terminus to the C-terminus). The intracellular signaling region of CD3-zeta is residues 22-164 of SEQ ID NO: 10. ITAM1 is located around amino acid residues 61-89, ITAM2 around amino acid residues 100-128, and ITAM3 around residues 131 -159. Thus, the modified CD3 zeta polypeptide may have any one of ITAM1, ITAM2, or ITAM3 inactivated, e.g. disrupted or deleted. Alternatively, the modified CD3 zeta polypeptide may have any two ITAMs inactivated, e.g. ITAM2 and ITAM3, or ITAM1 and ITAM2. Preferably, ITAM3 is inactivated, e.g. deleted. More preferably, ITAM2 and ITAM3 are inactivated, e.g. deleted, leaving ITAM1. For example, one modified CD3 zeta polypeptide retains only ITAM1 and the remaining CD3zeta domain is deleted (residues 90-164). As another example, ITAM1 is substituted with the amino acid sequence of ITAM3, and the remaining CD3zeta domain is deleted (residues 90-164). See, for example, Bridgeman et al., Clin. Exp. Immunol. 175(2): 258-67 (2014); Zhao et al., J. Immunol. 183(9): 5563-74 (2009); Maus et al., WO-2018/132506; Sadelain et al., WO-2019/133969, Feucht et al., Nat Med. 25(1):82-88 (2019).

Thus, in some aspects, the antigen binding molecule is linked to one or more cell signaling modules. In some embodiments, cell signaling modules include CD3 transmembrane domain, CD3 intracellular signaling domains, and/or other CD transmembrane domains. The CAR can also further include a portion of one or more additional molecules such as Fc receptor g, CD8, CD4, CD25, or CD16.

In some embodiments, upon ligation of the CAR, the cytoplasmic domain or intracellular signaling domain of the CAR activates at least one of the normal effector functions or responses of the corresponding non-engineered immune cell (typically a T cell). For example, the CAR can induce a function of a T cell such as cytolytic activity or T-helper activity, secretion of cytokines or other factors.

The CAR or other antigen-specific receptor can also be an inhibitory CAR (e.g. iCAR) and includes intracellular components that dampen or suppress a response, such as an immune response. Examples of such intracellular signaling components are those found on immune checkpoint molecules, including PD-1, CTLA4, LAG3, BTLA, OX2R, TIM-3, TIGIT, LAIR-1, PGE2 receptors, or EP2/4 Adenosine receptors including A2AR. In some aspects, the engineered cell includes an inhibitory CAR including a signaling domain of or derived from such an inhibitory molecule, such that it serves to dampen the response of the cell. Such CARs are used, for example, to reduce the likelihood of off-target effects when the antigen recognized by the activating receptor, e.g, CAR, is also expressed, or may also be expressed, on the surface of normal cells. *TCRs*

In some embodiments, the antigen-specific receptors include recombinant modified T cell receptors (TCRs) and/or TCRs cloned from naturally occurring T cells. Nucleic acid encoding the TCR can be obtained from a variety of sources, such as by polymerase chain reaction (PCR) amplification of naturally occurring TCR DNA sequences, followed by expression of antibody variable regions, followed by selecting for specific binding to antigen. In some embodiments, the TCR is obtained from T-cells isolated from a patient, or from cultured T-cell hybridomas. In some embodiments, the TCR clone for a target antigen has been generated in transgenic mice engineered with human immune system genes (e.g., the human leukocyte antigen system, or HLA). See, e.g., tumor antigens (see, e.g., Parkhurst et al. (2009) Clin Cancer Res. 15:169-180 and Cohen et al. (2005) J Immunol. 175:5799-5808. In some embodiments, phage display is used to isolate TCRs against a target antigen (see, e.g., Varela-Rohena et al. (2008) Nat Med. 14:1390-1395 and Li (2005) Nat Biotechnol. 23:349-354.

A "T cell receptor" or "TCR" refers to a molecule that contains a variable alpha and beta chains (also known as TCRα and TCRβ, respectively) or a variable gamma and delta chains (also known as TCRy and TCRδ, respectively) and that is capable of specifically binding to an antigen peptide bound to a MHC receptor. In some embodiments, the antigen-binding domain of the TCR binds its target antigen with KD affinity of about 1 × 10⁻⁷ or less, about 5 × 10⁻⁸ or less, about 1 × 10⁻⁸ or less, about 5 × 10⁻⁹ or less, about 1 × 10⁻⁹ or less, about 5 × 10⁻¹⁰ or less, about 1 × 10⁻¹⁰ or less, about 5 × 10⁻¹¹ or less, about 1 × 10⁻¹¹ or less, about 5 × 10⁻¹² or less, or about 1 × 10⁻¹² or less (lower numbers indicating greater binding affinity). In some embodiments, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules. In some embodiments, a TCR also can contain a constant domain, a transmembrane domain and/or a short cytoplasmic tail (see, e.g., Janeway et ah, Immunobiology: The Immune System in Health and Disease, 3rd Ed., Current Biology Publications, p. 4:33, 1997). For example, in some aspects, each chain of the TCR can possess one N-terminal immunoglobulin variable domain, one immunoglobulin constant domain, a transmembrane region, and a short cytoplasmic tail at the C-terminal end. In some embodiments, a TCR is associated with invariant proteins of the CD3 complex involved in mediating signal transduction. Unless otherwise stated, the term "TCR" should be understood to encompass functional TCR fragments thereof. The term also encompasses intact or full-length modified TCRs, including TCRs in the αβ form or γδ form. The term "TCR" also includes a TCR modified to include a VH and/or VL of an antibody.

Thus, for purposes herein, reference to a TCR includes any modified TCR or functional fragment thereof, such as an antigen-binding portion of a TCR that binds to a specific antigenic peptide bound in an MHC molecule, i.e. MHC-peptide complex. An "antigen binding portion" or "antigen-binding fragment" of a TCR, which can be used interchangeably, refers to a molecule that contains a portion of the structural domains of a TCR, but that binds the antigen (e.g. MHC-peptide complex) to which the full TCR binds. In some cases, an antigen-binding portion contains the variable domains of a TCR, such as variable alpha chain and variable beta chain of a TCR, sufficient to form a binding site for binding to a specific MHC-peptide complex, such as generally where each chain contains three complementarity determining regions.

In some embodiments, the variable domains of the TCR chains associate to form loops, or complementarity determining regions (CDRs) analogous to immunoglobulins, which confer antigen recognition and determine peptide specificity by forming the binding site of the TCR molecule and determine peptide specificity. Typically, like immunoglobulins, the CDRs are separated by framework regions (FRs) (see, e.g., Jores et al., Proc. Nat'l. Acad. Sci. U.S.A. 87:9138, 1990; Chothia et al., EMBO J. 7:3745, 1988; see also Lefranc et al., Dev. Comp. Immunol. 27:55, 2003). In some embodiments, CDR3 is the main CDR responsible for recognizing processed antigen, although CDR1 of the alpha chain has also been shown to interact with the N-terminal part of the antigenic peptide, whereas CDR1 of the beta chain interacts with the C-terminal part of the peptide. CDR2 is thought to recognize the MHC molecule. In some embodiments, the variable region of the beta chain can contain a further hypervariability (HV4) region.

In some embodiments, the TCR chains contain a constant domain. For example, like immunoglobulins, the extracellular portion of TCR chains (e.g., α-chain, β-chain) can contain two immunoglobulin domains, a variable domain {e.g., Vα or Vβ; typically amino acids 1 to 116 based on Kabat numbering Kabat et al., Sequences of Proteins of Immunological Interest, US Dept. Health and Human Services, Public Health Service National Institutes of Health, 1991 , 5th ed.) at the N-terminus, and one constant domain (e.g., α-chain constant domain or Cα or TRAC, typically amino acids 117 to 259 based on Kabat, β-chain constant domain or Cβ or TRBC, typically amino acids 117 to 295 based on Kabat) adjacent to the cell membrane. For example, in some cases, the extracellular portion of the TCR formed by the two chains contains two membrane-proximal constant domains, and two membrane-distal variable domains containing CDRs. The constant domain of the TCR domain contains short connecting sequences in which a cysteine residue forms a disulfide bond, making a link between the two chains. In some embodiments, a TCR may have an additional cysteine residue in each of the α and β chains such that the TCR contains two disulfide bonds in the constant domains.

In some embodiments, the TCR chains can contain a transmembrane domain. In some embodiments, the transmembrane domain is positively charged. In some cases, the TCR chains contain a cytoplasmic tail. In some cases, the structure allows the TCR to associate with other molecules like CD3. For example, a TCR containing constant domains with a transmembrane region can anchor the protein in the cell membrane and associate with invariant subunits of the CD3 signaling apparatus or complex.

Generally, CD3 is a multi-protein complex that can possess three distinct chains (gamma (γ), delta (δ), and epsilon (ε)) and the zeta-chain. For example, in mammals the complex can contain a CD3gamma chain, a CD3delta chain, two CD3epsilon chains, and a homodimer of CD3zeta chains. The CD3gamma chains are highly related cell surface proteins of the immunoglobulin superfamily containing a single immunoglobulin domain. The transmembrane regions of the CD3gamma, CD3delta, and CD3epsilon chains are negatively charged, which is a characteristic that allows these chains to associate with the positively charged T cell receptor chains and play a role in propagating the signal from the TCR into the cell. The intracellular tails of the CD3gamma, CD3delta, and CD3epsilon chains each contain a single conserved motif known as an immunoreceptor tyrosine-based activation motif or ITAM, whereas each CD3 zeta chain has three ITAMs. Generally, ITAMs are involved in the signaling capacity of the TCR complex. The CD3gamma, delta, epsilon and zeta chains together form what is known as the T cell receptor complex.

In some embodiments, modified TCR include recombinant HLA-independent (or non-HLA restricted) T cell receptors (referred to as "HI-TCRs") that bind to an antigen of interest in an HLA-independent manner. Such HI-TCRs comprise an antigen binding chain that comprises: (a) an antigen-binding domain as previously herein described that binds to an antigen in an HLA-independent manner, for example, an antigen-binding fragment of an immunoglobulin variable region; and (b) a constant domain that is capable of associating with (and consequently activating) a CD3ζ polypeptide. Because typically TCRs bind antigen in a HLA-dependent manner, the antigen-binding domain that binds in an HLA-independent manner must be heterologous. HLA independent (modified) TCRs are notably described in International Application No. WO 2019/157454. Such modified TCR, designated HI-TCR or HIT-CAR herein, can comprise (a) a first antigen-binding chain comprising an antigen-binding fragment of a heavy chain variable region (VH) of an antibody; and (b) a second antigen-binding chain comprising an antigen-binding fragment of a light chain variable region (VL) of an antibody; wherein the first and second antigen-binding chains each comprise a native or variant TRAC (constant region) or fragment thereof, or a native or variant TRBC (constant region) or fragment thereof. In some embodiments, at least one of the TRAC polypeptide and the TRBC polypeptide is endogenous, typically the TRAC polypeptide, and optionally one or both of the endogenous TRAC and TRBC polypeptides is inactivated.

In some designs, the HI-TCR comprises (a) a chimeric TCR alpha chain comprising a VH or fragment thereof fused to a native or variant TRAC or fragment thereof, optionally in which amino acids of the VH (or TRAC) are removed, and (b) a chimeric TCR beta chain comprising a VL or fragment thereof fused to a native or variant TRBC or fragment thereof, optionally in which amino acids of the VL (or TRBC) are removed. In other designs, the HI-TCR comprises (a) a chimeric TCR alpha chain comprising a VL or fragment thereof fused to a native or variant TRAC or fragment thereof, optionally in which amino acids of the VL (or TRAC) are removed, and (b) a chimeric TCR beta chain comprising a VH or fragment thereof fused to a native or variant TRBC or fragment thereof, optionally in which amino acids of the VH (or TRBC) are removed. In yet other designs, the HI-TCR comprises just a VH or fragment thereof fused to a native or variant TRAC or fragment thereof, or fused to a native or variant TRBC or fragment thereof, optionally in which amino acids of the VH (or TRAC or TRBC) are removed. HI-TCR (HIT-CAR) are described in Int'l Pat. Pub. No. WO 2019/157454, incorporated by reference herein in its entirety. Yet other modified TCRs are disclosed in Int'l Pat. Pub. No. WO 2018/067993, incorporated herein by reference in its entirety, and in Baeuerle, et al. Synthetic TRuC receptors engaging the complete T cell receptor for potent anti-tumor response. Nat Commun 10, 2087 (2019). For example, any one or more, or two or more, of the alpha, beta, gamma or epsilon chains (e.g. intracellular and optionally transmembrane domains thereof) may be fused to an antibody variable region, e.g., VH and/or VL, or an scFv.

In some embodiments, the nucleic acid encoding the heterologous antigen-binding domain (e.g., VH or variant or fragment thereof, or VL or variant or fragment thereof) is inserted into the endogenous TRAC locus and/or TRBC locus of the immune cell. Optionally, the nucleic acid encoding the chimeric TCR alpha (or beta) chain is operatively linked to an endogenous promoter of the T-cell receptor such that its expression is under control of the endogenous promoter. The insertion of the nucleic acid sequence can also inactivate or disrupt the endogenous expression of a TCR comprising a native TCR alpha chain and/or a native TCR beta chain. The insertion of the nucleic acid sequence may reduce endogenous TCR expression by at least about 75%, 80%, 85%, 90% or 95%.

Other examples of antigen-specific receptors, including CARs and recombinant modified TCRs, as well as methods for engineering and introducing the receptors into cells, include those described, for example, in international patent application publication numbers WO-2000/014257, WO-2013/126726, WO-2012/129514, WO-2014/031687, WO-2013/166321, WO-2013/071154, WO-2013/123061 U.S. patent application publication numbers US-2002131960, US-2013287748, US-20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191 , 8,324,353, and 8,479,118, and European patent application number EP2537416, and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen-specific receptors include a CAR as described in U.S. Patent No.: 7,446,190, and those described in International Patent Application Publication No.: WO-2014/055668 A1.

### Co-stimulatory receptors

The cells of the disclosure with modified SUV39H1 expression may further comprise at least one or at least two exogenous co-stimulatory ligands. Co-stimulatory ligands include CD80, CD86, 4-1BBL, CD275, CD40L, OX40L or any combination thereof. In some embodiments, the co-stimulatory ligand is CD80 or 4-1BBL.

In some embodiments, the cells comprise at least one or at least two co-stimulatory receptors. Such co-stimulatory receptors include chimeric receptors comprising a co-stimulatory ligand fused to at least one or at least two co-stimulatory molecule(s). Co-stimulatory ligands include CD80, CD86, 4-1BBL, CD275, CD40L, OX40L or any combination thereof. In some embodiments, the co-stimulatory ligand is CD80 or 4-1BBL. Example co-stimulatory molecules are CD28, 4-1BB, 0X40, ICOS, DAP-10, CD27, CD40, NKG2D, CD2, or any combination thereof. In some embodiments, the chimeric receptor comprises a first co-stimulatory molecule that is 4-1BB and a second co-stimulatory molecule that is CD28.

In some embodiments, the cell comprises a co-stimulatory receptor comprising the extracellular domain of CD80, transmembrane domain of CD80, and an intracellular 4-1BB domain. Example co-stimulatory ligands, molecules and receptors (or fusion polypeptides) are described in Int'l Pat. Pub. No. WO-2021/016174, incorporated by reference herein in its entirety.

The cells of the disclosure with modulated SUV39H1 expression may also comprise T-cell specific engagers, such as BiTEs, or bispecific antibodies that bind not only the desired antigen but also an activating T-cell antigen such as CD3 epsilon. In some embodiments, the BiTe comprises an antigen-binding domain, e.g. scFv, linked to a T-cell recognizing domain, e.g., heavy variable domain and/or light variable domain of an anti-CD3 antibody.

### Antigens

Antigens include antigens associated with diseases or disorders, including proliferative, neoplastic, and malignant diseases and disorders, more particularly cancers. Infectious diseases and autoimmune, inflammatory or allergic diseases are also contemplated.

The cancer may be a solid cancer (solid tumor) or a "liquid tumor" such as cancers affecting the blood, bone marrow and lymphoid system, also known as tumors of the hematopoietic and lymphoid tissues, which notably include leukemia and lymphoma. Liquid tumors include for example acute myelogenous leukemia (AML), chronic myelogenous leukemia (CML), acute lymphocytic leukemia (ALL), and chronic lymphocytic leukemia (CLL), (including various lymphomas such as mantle cell lymphoma, non-Hodgkins lymphoma (NHL) Central nervous system lymphoma (CNSL), adenoma, squamous cell carcinoma, laryngeal carcinoma, gallbladder and bile duct cancers, cancers of the retina such as retinoblastoma).

Solid cancers (also called solid tumors) notably include cancer affecting an organ, optionally colon, rectum, skin, endometrium, lung (including non-small cell lung carcinoma), uterus, bones (such as Osteosarcoma, Chondrosarcomas, Ewing's sarcoma, Fibrosarcomas, Giant cell tumors, Adamantinomas, and Chordomas), liver, kidney, esophagus, stomach, bladder, pancreas, cervix, brain (such as Meningiomas, Glioblastomas, Lower-Grade Astrocytomas, Oligodendrocytomas, Pituitary Tumors, Schwannomas, and Metastatic brain cancers), ovary, breast (including triple negative breast cancer), head and neck region, testis, prostate or the thyroid gland.

Cancers include cancers affecting the blood, bone marrow and lymphoid system as described above. In some embodiments, the cancer is, or is associated, with multiple myeloma. Antigens associated with multiple myeloma include CD38, CD138, and/or CS-1. Other exemplary multiple myeloma antigens include CD56, TIM-3, CD33, CD123, and/or CD44.

Diseases also encompass infectious diseases or conditions, such as, but not limited to, viral, retroviral, bacterial, protozoal or parasitic, infections, or viral infections caused by, e.g., human immunodeficiency virus (HIV), Cytomegalovirus (CMV), Epstein-Barr virus (EBV), adenovirus, BK polyomavirus, hepatitis virus, such as hepatitis B, hepatitis C, hepatitis D, hepatitis E.

In some embodiments the extracellular antigen-binding domain binds to any of the tumor neoantigenic peptides disclosed in Int'l Pat. Pub. No. WO 2021/043804, incorporated by reference herein in its entirety. For example, the antigen-binding domain binds to any of the peptides of F or to a neoantigenic peptide comprising at least 8, 9, 10, 11 or 12 amino acids that is encoded by a part of an open reading frame (ORF) of any of the fusion transcript sequences of any one of SEQ ID NO: 118-17492 of WO 2021/043804.

Diseases also encompass autoimmune or inflammatory diseases or conditions, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease multiple sclerosis, asthma, and/or diseases or conditions associated with transplant. In such circumstances, a T-regulatory cell may be the cell in which SUV39H1 is inhibited.

In some embodiments, the antigen is a polypeptide. In some embodiments, it is a carbohydrate or other molecule. In some embodiments, the antigen is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells. In some such embodiments, a multitargeting and/or gene disruption approach as provided herein is used to improve specificity and/or efficacy.

In some embodiments, the antigen is a universal tumor antigen. The term "universal tumor antigen" refers to an immunogenic molecule, such as a protein, that is, generally, expressed at a higher level in tumor cells than in non-tumor cells and also is expressed in tumors of different origins. In some embodiments, the universal tumor antigen is expressed in more than 30%, 40%, 50%, 60%, 70%, 75%, 80%, 85%, 90% or more of human cancers. In some embodiments, the universal tumor antigen is expressed in at least three, at least four, at least five, at least six, at least seven, at least eight or more different types of tumors. In some cases, the universal tumor antigen may be expressed in non-tumor cells, such as normal cells, but at lower levels than it is expressed in tumor cells. In some cases, the universal tumor antigen is not expressed at all in non-tumor cells, such as not expressed in normal cells. Exemplary universal tumor antigens include, for example, human telomerase reverse transcriptase (hTERT), survivin, mouse double minute 2 homolog (MDM2), cytochrome P450 1 B1 (CYP1 B), HER2/neu, p95HER2, Wilms tumor gene 1 (WT1), livin, alphafetoprotein (AFP), carcinoembryonic antigen (CEA), mucin 16 (MUC16), MUC1, prostate-specific membrane antigen (PSMA), p53 or cyclin (Dl). Peptide epitopes of tumor antigens, including universal tumor antigens, are known in the art and, in some aspects, can be used to generate MHC-restricted antigen-specific receptors, such as TCRs or TCR-like CARs (see e.g. published PCT application No. WO-2011/009173 or WO-2012/135854 and published U.S. application No. US-20140065708).

In some embodiments, the cancer is, or is associated, with overexpression of HER2 or p95HER2. p95HER2 is a constitutively active C-terminal fragment of HER2 that is produced by an alternative initiation of translation at methionine 611 of the transcript encoding the full-length HER2 receptor. HER2 or p95HER2 has been reported to be overexpressed in breast cancer, as well as gastric (stomach) cancer, gastroesophageal cancer, esophageal cancer, ovarian cancer, uterine endometrial cancer, cervix cancer, colon cancer, bladder cancer, lung cancer, and head and neck cancers. Patients with cancers that express the p95HER2 fragment have a greater probability of developing metastasis and a worse prognosis than those patients who mainly express the complete form of HER2. Saez et al., Clinical Cancer Research, 12:424-431 (2006).

Other antigens include orphan tyrosine kinase receptor ROR1, tEGFR, Her2, p95HER2, LI-CAM, CD19, CD20, CD22, mesothelin, CEA, Claudin 18.2, hepatitis B surface antigen, anti-folate receptor, CD23, CD24, CD30, CD33, CD38, CD44, CD70, EGFR, EGP-2, EGP-4, EPHa2, ErbB2, 3, or 4, FBP, FcRH5 fetal acetylcholine e receptor, GD2, GD3, HMW-MAA, IL-22R-alpha, IL-13R-alpha2, kdr, kappa light chain, Lewis Y, Ll-cell adhesion molecule, MAGE-A1 , mesothelin, MUC1 , MUC16, PSCA, NKG2D Ligands, NY-ESO-1 , MART-1 , gplOO, oncofetal antigen, ROR1 , TAG72, VEGF-R2, carcinoembryonic antigen (CEA), prostate specific antigen, PSMA, Her2/neu, p95HER2, estrogen receptor, progesterone receptor, ephrinB2, CD 123, CS-1 , c-Met, GD-2, and MAGE A3, CE7, Wilms Tumor 1 (WT-1 ), a cyclin, such as cyclin A1 (CCNA1), and/or molecules expressed by HIV, HCV, HBV or other pathogens.

### Therapeutic uses

The cells of the disclosure may be used in adoptive cell therapy (notably adoptive T cell therapy or adoptive NK cell therapy). In some embodiments, the use is in the treatment of cancer in a subject in need thereof, but uses also include the treatment of infectious diseases and autoimmune, inflammatory or allergic diseases. In some embodiments, the subject is suffering from a cancer or at risk of suffering from a cancer.

The cells of the disclosure are particularly beneficial when administered to treat a chronic disease, such as a chronic infectious disease, or when administered to treat refractory, relapsed or resistant cancer.

In some embodiments, the patient exhibits a cancer relapse or is likely to exhibit a cancer relapse. In some embodiments, the patient exhibits cancer metastasis or is likely to exhibit cancer metastasis. In some embodiments, the patient has not achieved sustained cancer remission after one or more prior cancer therapies. In some embodiments, the patient suffers from a cancer that is resistant or nonresponsive to one or more prior cancer therapies. In some embodiments, the patient suffers from a refractory cancer. In some embodiments, the patient is likely to exhibit a response to cell therapy that is not durable. In some embodiments, the patient is ineligible for immune checkpoint therapy or did not respond to immune checkpoint therapy. In some embodiments, the patient is ineligible for treatment with high dose of chemotherapy and/or is ineligible for treatment with high adoptive cell therapy doses.

In such methods, one or more types of modified immune cells as described herein are administered to a subject in need thereof, in an amount effective to treat the disease or disorder. For example, cells expressing one or more antigen-specific receptors as described herein (including with reduced SUV39H1 activity and optionally comprising a CAR with a single active ITAM as described herein) are administered at a dose effective to treat the disease or disorder associated with the antigen(s). Treatment of any of the diseases listed above under the "Antigen" section is contemplated.

The cells may be administered at certain doses. For example, the immune cells (e.g., T cells or NK cells) in which SUV39H1 has been inhibited may be administered to adults at doses of less than about 10⁸ cells, less than about 5 × 10⁷ cells, less than about 10⁷ cells, less than about 5 × 10⁶ cells, less than about 10⁶ cells, less than about 5 × 10⁵ cells or less than about 10⁵ cells. The dose for pediatric patients may be about 100-fold less. In alternative embodiments, any of the immune cells (e.g. T-cells) described herein may be administered to patients at doses ranging from about 10⁵ to about 10⁹ cells, or about 10⁵ to about 10⁸ cells, or about 10⁵ to about 10⁷ cells, or about 10⁶ to about 10⁸ cells.

The subject (i.e. patient) is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent. In some examples, the patient or subject is a validated animal model for disease, adoptive cell therapy, and/or for assessing toxic outcomes such as cytokine release syndrome (CRS). In some embodiments, said subject has a cancer, is at risk of having a cancer, or is in remission of a cancer.

In some embodiments, the cells or compositions are administered to the subject, such as a subject having or at risk for a cancer or any one of the diseases as mentioned above. In some aspects, the methods thereby treat, e.g., ameliorate one or more symptom of, the disease or condition, such as with reference to cancer, by lessening tumor burden in a cancer expressing an antigen recognized by the engineered cell.

Methods for administration of cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31 (10): 928-933; Tsukahara et al. (2013) Biochem Biophys Res Commun 438(1): 84-9; Davila et al. (2013) PLoS ONE 8(4): e61338.

Administration of at least one cell according to the disclosure to a subject in need thereof may be combined with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cell populations are administered prior to the one or more additional therapeutic agents. In some embodiments, the cell populations are administered after to the one or more additional therapeutic agents.

With reference to cancer treatment, a combined cancer treatment can include but is not limited to cancer chemotherapeutic agents, cytotoxic agents, hormones, anti-angiogens, radiolabelled compounds, immunotherapy, surgery, cryotherapy, and/or radiotherapy.

Conventional cancer chemotherapeutic agents include alkylating agents, antimetabolites, anthracyclines, topoisomerase inhibitors, microtubule inhibitors and B- raf enzyme inhibitors.

Alkylating agents include the nitrogen mustards (such as mechlorethamine, cyclophosphamide, ifosfamide, melphalan and chlorambucil), ethylenamine and methylenamine derivatives (such as altretamine, thiotepa), alkyl sulfonates (such as busulfan), nitrosoureas (such as carmustine, lomustine, estramustine), triazenes (such as dacarbazine, procarbazine, temozolomide), and platinum-containing antineoplastic agents (such as cisplatin, carboplatin, oxaliplatin).

Antimetabolites include 5-fluorouracil (5-FU), 6-mercaptopurine (6-MP), Capecitabine (Xeloda^{®}), Cytarabine (Ara-C^{®}), Floxuridine, Fludarabine, Gemcitabine (Gemzar^{®}), Hydroxyurea, Methotrexate, Pemetrexed (Alimta^{®}).

Anthracyclines include Daunorubicin, Doxorubicin (Adriamycin^{®}), Epirubicin. Idarubicin. Other anti-tumor antibiotics include Actinomycin-D, Bleomycin, Mitomycin-C, Mitoxantrone.

Topoisomerase inhibitors include Topotecan, Irinotecan (CPT-11), Etoposide (VP-16), Teniposide or Mitoxantrone.

Microtubule inhibitors include Estramustine, Ixabepilone, the taxanes (such as Paclitaxel, Docetaxel and Cabazitaxel), and the vinca alkaloids (such as Vinblastine, Vincristine, Vinorelbine, Vindesine and Vinflunine)

B-raf enzyme inhibitors include vemurafenib (Zelboraf), dabrafenib (Tafinlar), and encorafenib (Braftovi).

Immunotherapy includes but is not limited to immune checkpoint modulators (i.e. inhibitors and/or agonists), cytokines, immunomodulating monoclonal antibodies, cancer vaccines.

Preferably, administration of cells in an adoptive T cell therapy according to the disclosure is combined with administration of immune checkpoint modulators. Examples include inhibitors of (e.g. antibodies that bind specifically to and inhibit activity of) PD-1, CTLA4, LAG 3, BTLA, OX2R, TIM-3, TIGIT, LAIR-1, PGE2 receptors, and/or EP2/4 Adenosine receptors including A2AR. Preferably, the immune checkpoint modulators comprise anti-PD-1 and/or anti-PDL-1 inhibitors (e.g., anti-PD-1 and/or anti-PDL-1 antibodies).

The present disclosure also relates to the use of a composition comprising the cells as herein described for the manufacture of a medicament for treating a cancer, an infectious disease or condition, an autoimmune disease or condition, or an inflammatory disease or condition in a subject.

### EXAMPLES

### Example 1: SUV39H1 KO CAR T cells better reject established solid tumors.

SUV39H1 KO CAR T cells were generated via lentiviral transduction of anti-CD19 CARs (custom order lentiviral particles, Flash Therapeutics) and CrispR/Cas9 mediated inactivation of SUV39H1 in healthy donor derived T cells. Efficient inactivation of SUV39H1 was achieved in cells treated with single SUV39H1 locus-targeting gRNAs (Integrated DNA Technologies, or Synthego) compared to Mock, with no differences in CAR expression. Mock cells thus expressed anti-CD19 CAR but were not treated with single SUV39H1 locus-targeting gRNAs to inactivate SUV39H1. To investigate the effect of SUV39H1 inactivation on CAR T cell antitumor efficacy, a xenogeneic model with orthotopic lung tumors was setup. A549 lung adenocarcinoma cells were generated to ectopically express luciferase and CD19 as a molecular target. These cells were then specifically targeted by anti-CD 19-CAR expressing T cells. Injection of A549-CD19-luc cells in the tail vein resulted in their delivery to the lung, where their growth was monitored by bioluminescence (IVIS, Perkin Elmer, or Photolmager, Biospace Lab) following intraperitoneal injections of 150 mg/kg D-Luciferin (D-Luciferin potassium salt, Perkin Elmer).

The effect of SUV39H1 inactivation on CAR T cell antitumor efficacy has been illustrated using an anti-CD19 CAR that has the following architecture: anti-CD19-scFv - CD8 (hinge and transmembrane) - 4-1BB (intracellular signaling domain) - CD3zeta (intracellular signaling domain) (referenced herein as "19-BBz"), described in Maude et al, N Eng J Med, 2014. Mice were first injected with A549-CD19-luc cells and, twenty-one days later, they were infused with either 3×10⁵ Mock or 3×10⁵ SUV39H1 KO 19-BBz CAR T cells (Figure 1A). As previously shown in Int'l. Pat. Pub. No. WO 2018/234370, SUV39H1 KO CAR T cells demonstrated faster and more long-lasting rejection of A549 orthotopic tumors (Figure 1B).

### Example 2: Use of a CAR construct including CD3z modified intracellular domain (1XX construct) further improves anti-tumor efficacy.

The effect of SUV39H1 inactivation on CAR T cell antitumor efficacy was tested using a different CAR structure, including a CD3z intracellular domain with inactivated ITAM2 and ITAM3). The 19-28z-1XX from Feucht et al., (Nat Med 2019) was thus expressed in SUV39H1 KO T cells. This 19-28z-1XX has the following architecture: anti-CD19-scFv - CD28 (hinge, transmembrane and intracellular signaling domain) - CD3zeta-1XX (intracellular signaling domain where ITAM2 and ITAM3 are inactivated). Mock cells expressed 19-28z-1XX CAR but were not treated to inactivate SUV39H1. Mice were first injected with A549-CD19-luc cells and, twenty-one days later, they are infused with either 5×10⁴ Mock or 5×10⁴ SUV39H1 KO 19-28z-1XX CAR T cells (Figure 2A). As previously described in Int'l. Pat. Pub. No. WO 2021/013950, the combination of SUV39H1 KO with 1XX CAR provides superior effects. SUV39H1 KO 1 XX CAR T cells demonstrated stronger rejection of A549 orthotopic tumors (Figure 2C) as compared to Mock 1XX CAR T cells (Figure 2B). Moreover, 5 out of 7 mice treated with Mock CAR T cells showed persistent tumor growth, in contrast to only 2 out of 5 mice treated with SUV39H1 KO CAR T cells. Overall, it was shown that the combination of 1XX CAR construct (e.g. 19-28z-1XX) and SUV39H1 inactivation was efficacious at the lowest dose tested (5×10⁴ cells), in contrast to the combination of SUV39H1 KO and CAR constructs with the native CD3z ITAMs (e.g. 19-BBz), which did not control A549-CD19 tumors at a dose of 10⁵ cells (data not shown). Thus, replacing a CAR having three ITAMs with a 1XX CAR having a single active ITAM was able to convert a lower, ineffective dose of cells into a therapeutically effective dose of cells in a solid tumor model.

### Example 3: SUV39H1 inactivation increases long-term CAR T cells persistency in peripheral organs.

In order to study persistence of CAR T cells in vivo in peripheral organs, a higher dose (9×10⁵ cells) of either Mock or SUV39H1 KO 19-BBz CAR T cells was infused in A549-CD19 tumor bearing NSG mice. See Figures 3A (Mock) and 3B (SUV39H1 KO). Both Mock and SUV39H1 KO CAR T cells were able to reject established tumors at this dose (Figures 3A and 3B, respectively). Mice were sacrificed at different time points: a) 8 days post CAR T cell infusion, the peak of the immune response, and b) 28 days post CAR T cell infusion, when there was no residual tumor left. Immune cells were antibody-stained and then analysed by flow cytometry in a FACS analyser (BD LSRII). Figures 3C and 3D show CAR T cell numbers in the lungs and spleens, respectively, at day 8 and day 28. While at Day 8 there were similar numbers of either Mock or SUV39H1 KO CAR T cells in the lungs and spleens of the mice tested (approximately 1 × 10⁵ for Mock and SUV39H1 KO), the number of Mock cells decreased dramatically in the spleen (approximately 1 × 10³) by Day 28 (Figure 3D). Therefore, SUV39H1 KO CAR T cells persist more than 10-fold better in the spleens of tumor bearing mice following tumor eradication.

The above effect could be due to better initial expansion of SUV39H1 or better overall persistence, or both. A lower dose (7.5×10⁵ cells) of either Mock or SUV39H1 KO 19-BBz CAR T cells was tested in mice, 10 days after tumor cell injection. See Figures 3E (Mock) and 3F (SUV39H1 KO). Both Mock and SUV39H1 KO CAR T cells were able to reject established tumors at this dose (Figures 3E and 3F, respectively). CAR T cell numbers were measured in peripheral blood of infused mice at different time points: a) 8 days post CAR T cell infusion, the peak of the immune response, and b) 63 days post CAR T cell infusion, when there was no residual tumor left (Figure 3G). SUV39H1 KO CAR T cells showed stronger expansion in peripheral blood on Day 8 compared to Mock cells. On Day 63, Mock CAR T cells have dramatically decreased (approximately 1 × 10²) while SUV39H1 KO CAR persist close to the initial expansion levels (approximately 1 × 10⁴), an approximately 100-fold difference. SUV39H1 KO CAR T cells thus demonstrate both stronger initial expansion and better overall persistence.

### Example 4: SUV39H1 KO 1XX CAR T cells demonstrate stronger long-term persistence in peripheral organs.

A superior effect on long term peripheral persistence has further been observed using the 1XX CAR construct (19-28z-1XX) with SUV39H1 KO, compared to Mock, over an even longer time period of 90 days after injection. A dose of 7.5×10⁵ cells of either Mock or SUV39H1 KO 19-BBz CAR T cells was infused in A549-CD19 tumor bearing NSG mice. Both Mock and SUV39H1 KO CAR T cells were able to reject established tumors at this dose. At Day 90 post CAR T cell infusion, mice were sacrificed, and the organs were analysed for CAR T cell infiltration. SUV39H1 KO 1XX CAR T cells were more abundant in both the spleens and lungs of sacrificed mice.

Therefore, SUV39H1 KO 1XX CAR T cells demonstrate stronger long-term persistence in peripheral organs following tumor eradication.

### Example 5: SUV39H1 KO CAR T cells better protect against multiple tumor rechallenges

A significant drawback of current CAR T cell therapies is the inability to control tumor relapses. In the A549-CD19 xenogeneic model, this was tested by re-injecting tumor cells in the mice following primary tumor rejection by CAR T cells and then following tumor growth by bioluminescence.

As shown in Figure 4A, mice were first injected with A549-CD19-luc cells (D0) and ten days later, they were infused with either Mock or SUV39H1 KO 19-BBz CAR T cells. Two doses were tested: 2.5×10⁵ (Figure 4B) and 7.5×10⁵ CAR T cells (Figure 4C). At these doses, both Mock and SUV39H1 KO CAR T cells were able to reject primary tumors (Figures 4B, 4C). Tumor rechallenges were performed three times for both CAR T doses for three consecutive weeks, on Days 95, 102, and 109 post initial tumor infusion (85, 92, and 99 post CAR T infusion). At the lower CAR T cell dose of 2.5×10⁵ cells, neither Mock nor SUV39H1 CAR T cells were able to control tumor relapses following rechallenge of the mice (Figure 4B). However, at the dose of 7.5×10⁵ CAR T cells, SUV39H1 KO cells were able to completely control rechallenges, in contrast to Mock cells which did not (Figure 4C). These results provide strong evidence that, SUV39H1 KO CAR T cells (e.g.,19-BBz CAR T cells) are superior at protecting against tumor relapses.

The combination of 19-28z-1XX and SUV39H1 inactivation was also tested in a rechallenge model (schematic depicted in Figure 5A). Briefly, mice were first injected with A549-CD19-luc cells (D-21), and twenty-one days later, they were infused with either Mock 19-28z-1XX CAR T cells (Figure 5B) or SUV39H1 KO 19-28z-1XX CAR T cells (Figure 5C), in both cases at a dose of 2.5×10⁵ cells (D0). Both Mock and SUV39H1 KO CAR T cells were able to reject primary tumors (Figures 5B, 5C). Tumor rechallenges were performed seven times for seven consecutive weeks, on Days 45, 52, 59, 66, 73, 80, and 87 post CAR T infusion. SUV39H1 KO 19-28z-1XX CAR T cells demonstrate complete control of tumor rechallenges (no mice have relapses), in contrast to Mock cells, where 3 out 7 mice show tumor relapses following rechallenge (Figures 5B, 5C). Therefore, SUV39H1 KO + 1XX CAR T cells are superior at protecting against tumor relapses. Notably, this dose of 2.5×10⁵ cells was ineffective when tested for SUV39H1 KO CAR T cells expressing conventional CARs comprising three ITAMs.

Overall, these results show that the combination of 1XX CAR construct (e.g. 19-28z-1XX) and SUV39H1 inactivation is efficacious at a lower dose (e.g. 2.5×10⁵ cells) in contrast to SUV39H1 KO cells expressing CAR constructs with the native CD3z ITAMs (e.g. 19-BBz) cells, and against even more tumor rechallenges. Thus, replacing a CAR having three ITAMs with a 1XX CAR having a single active ITAM was able to convert a lower, ineffective dose of cells into a therapeutically effective dose of cells in a 7x tumor rechallenge model that mimics a relapsing solid tumor.

All patents, patent applications, and publications are herein incorporated by reference in their entirety for all purposes and to the same extent as if each individual publication was specifically and individually indicated to be incorporated by reference in its entirety for any and all purposes.

While the foregoing articles and methods of this disclosure have been described in terms of preferred embodiments, and optional features, it will be apparent to those skilled in the art that variations or combinations may be applied without departing from the spirit and scope of the disclosure. Such variations and combinations are intended to be within the meaning and range of the disclosure as defined by the claims. The breadth and scope of the present disclosure should not be limited by any of the above-described exemplary aspects but should be defined only in accordance with the following claims and their equivalents. The terms and expressions which have been employed are used as terms of description and not of limitation, and there is no intention that in the use of such terms and expressions of excluding any equivalents of the features shown and described or portions thereof.

## Claims

1. A modified immune cell with reduced SUV39H1 activity, for use in the treatment of a patient suffering from a refractory, relapsed or resistant cancer or suffering from a chronic infectious disease, wherein the cell expresses one or more engineered antigen-specific receptors that bind an antigen associated with the cancer or the chronic infectious disease.

2. A modified immune cell for use according to claim 1 wherein the patient suffers from a cancer, wherein said patient exhibits a cancer relapse or is likely to exhibit a cancer relapse, exhibits cancer metastasis or is likely to exhibit cancer metastasis, has not achieved sustained cancer remission after one or more prior cancer therapies, suffers from a cancer that is resistant or nonresponsive to one or more prior cancer therapies, suffers from a refractory cancer, is likely to exhibit a response to cell therapy that is not durable, is ineligible for immune checkpoint therapy or did not respond to immune checkpoint therapy, is ineligible for treatment with high dose of chemotherapy and/or is ineligible for treatment with high adoptive cell therapy doses.

3. A modified immune cell for use according to any of claim 1 or 2, wherein the antigen is orphan tyrosine kinase receptor ROR1, tEGFR, Her2, p95HER2, Ll-CAM, CD19, CD20, CD22, mesothelin, CEA, Claudin 18.2, hepatitis B surface antigen, anti-folate receptor, CD23, CD24, CD30, CD33, CD38, CD44, EGFR, EGP-2, EGP-4, CD70, EPHa2, ErbB2, 3, or 4, FcRH5, FBP, fetal acethycholine e receptor, GD2, GD3, HMW-MAA, IL-22R-alpha, IL-13R-alpha2, kdr, kappa light chain, BCMA, Lewis Y, MAGE-A1, mesothelin, MUC1, MUC16, PSCA, NKG2D Ligands, NY-ESO-1, MART-1, gplOO, oncofetal antigen, TAG72, VEGF-R2, carcinoembryonic antigen (CEA), prostate specific antigen (PSMA), estrogen receptor, progesterone receptor, ephrinB2, CD 123, CS-1, c-Met, GD-2, MAGE A3, CE7, or Wilms Tumor 1 (WT-1); or optionally any of the tumor neoantigenic peptides disclosed in Int'l Pat. Pub. No. WO 2021/043804.

4. A modified immune cell for use according to any of claims 1 to 3 wherein the cancer is a myeloid or a lymphoid cancer.

5. A modified immune cell for use according to any of claims 1 to 3 wherein the cancer is a solid tumor.

6. A modified immune cell for use according to claim 5 wherein the solid tumor is a cancer affecting an organ, optionally colon, rectum, skin, endometrium, lung (including non-small cell lung carcinoma), uterus, bones (such as Osteosarcoma, Chondrosarcomas, Ewing's sarcoma, Fibrosarcomas, Giant cell tumors, Adamantinomas, and Chordomas), liver, kidney, esophagus, stomach, bladder, pancreas, cervix, brain (such as Meningiomas, Glioblastomas, Lower-Grade Astrocytomas, Oligodendrocytomas, Pituitary Tumors, Schwannomas, and Metastatic brain cancers), ovary, breast (such as triple negative, or luminal breast cancer), head and neck region, testis, prostate or the thyroid gland.

7. A modified immune cell for use according to claim 1 wherein the patient suffers from a chronic viral infection, and optionally wherein the immune cell allows long term infection control and/or increased viral clearance.

8. A modified immune cell for use according to claim 7 wherein the chronic viral infection is caused by human immunodeficiency virus (HIV), Cytomegalovirus (CMV), Epstein-Barr virus (EBV), adenovirus, BK polyomavirus, hepatitis virus, such as hepatitis B, hepatitis C, hepatitis D, hepatitis E.

9. A modified immune cell for use according to any one of claims 1 to 8, wherein the SUV39H1 activity is inhibited.

10. A modified immune cell for use according to claim 9 which has been contacted with an exogenous SUV39H1 inhibitor, optionally a nucleic acid inhibitor of SUV39H1.

11. A modified immune cell for use according to claim 10, wherein the exogenous SUV39H1 inhibitor is (a) a dominant negative inhibitor, or (b) an RNAi, shRNA, ribozyme or antisense oligonucleotide complementary to a fragment of the SUV39H1 gene, or (c) an epipolythiodioxopiperazine (ETP) class of SUV39H1 inhibitor.

12. A modified immune cell for use according to any one of claims 1 to 8, wherein the cell's SUV39H1 gene comprises one or more mutations that results in a deleted or non-functional SUV39H1 protein, or a SUV39H1 protein with reduced activity.

13. A modified immune cell for use according to any one of claims 1 to 12, wherein the antigen-specific receptor is a modified TCR.

14. A modified immune cell for use according to any one of claims 1 to 12, wherein the antigen-specific receptor is a chimeric antigen receptor (CAR).

15. A modified immune cell for use according to any one of claims 1 to 14, wherein the cell expresses at least one antigen-specific receptor having an intracellular signaling domain wherein one or two immunoreceptor tyrosine-based activation motifs (ITAMs) are inactivated, optionally wherein the antigen-specific receptor comprises a single active ITAM domain.

16. A modified immune cell for use according to any one of claims 1 to 14, wherein the antigen-specific receptor is a chimeric antigen receptor (CAR) comprising:
a) an extracellular antigen-binding domain that specifically binds an antigen,
b) a transmembrane domain,
c) optionally one or more costimulatory domains, and
d) an intracellular signaling domain wherein one or two immunoreceptor tyrosine-based activation motifs (ITAMs) are inactivated, optionally wherein the antigen-specific receptor comprises a single active ITAM domain, optionally an intracellular domain comprising a modified CD3zeta intracellular signaling domain in which ITAM2 and ITAM3 have been inactivated.

17. A modified immune cell for use according to any one of claims 1 to 16, wherein the antigen-specific receptor comprises an extracellular antigen-binding domain which is an scFv; or an antibody heavy chain region (VH) and/or an antibody variable region (VL); or optionally a bispecific or trispecific antigen-binding domain.

18. A modified immune cell for use according to any one of claims 1 to 17, wherein the transmembrane domain is from CD28, CD8 or CD3-zeta, or a fragment thereof.

19. A modified immune cell for use according to any one of claims 1 to 18, wherein the one or more costimulatory domains are selected from the group consisting of: 4-1BB (CD137), CD28, CD27, ICOS, OX40 (CD134) and DAP10; DAP12, 2B4, CD40, FCER1G and/or GITR (AITR), or an active fragment thereof.

20. A modified immune cell for use according to any one of claims 1 to 16, wherein the antigen-specific receptor comprises:
a) an extracellular antigen-binding domain that specifically binds an antigen, optionally comprising an antibody heavy chain variable region and/or an antibody light chain variable region, and is optionally bispecific or trispecific;
b) a transmembrane domain, optionally comprising a fragment of transmembrane domain of alpha, beta or zeta chain of the T-cell receptor, CD28, CD3 epsilon, CD45, CD4, CD5, CD8, CD9, CD16, CD22, CD33, CD37, CD64, CD80, CD86, CD34, CD137, or CD154, NKG2D, OX40, ICOS, 2B4, DAP10, DAP12, CD40; and
c) optionally one or more co-stimulatory domains from 4-1BB, CD28, ICOS, OX40, DAP10 or DAP12, 2B4, CD40, FCER1G, or an active fragment thereof;
d) an intracellular signaling domain comprising an intracellular signaling domain from CD3zeta, FcR gamma, FcR beta, CD3 gamma, CD3 delta, CD3 epsilon, CDS, CD22, CD79a, CD79b, or CD66d, 2B4, or an active fragment thereof.

21. A modified immune cell for use according to any one of claims 1 to 16 wherein the antigen-specific receptor is a chimeric antigen receptor (CAR) comprising:
a) an extracellular antigen-binding domain, optionally an scFv,
b) a transmembrane domain, optionally from CD28, CD8 or CD3-zeta,
c) one or more co-stimulatory domains, optionally from 4-1 BB, CD28, ICOS, OX40 or DAP10, and
d) an intracellular signaling domain from CD3zeta, optionally in which ITAM2 and ITAM3 have been inactivated.

22. A modified immune cell for use according to any one of claims 1 to 16, wherein the antigen-specific receptor is a modified TCR that comprises a heterologous extracellular antigen-binding domain that specifically binds an antigen, optionally comprising an antibody heavy chain variable region and/or an antibody light chain variable region, and is optionally bispecific or trispecific.

23. A modified immune cell for use according to any one of claims 1 to 16, wherein the antigen-specific receptor is a modified TCR that comprises a fragment of an alpha, beta, gamma or epsilon chain and a heterologous extracellular antigen-binding domain that specifically binds an antigen, optionally comprising an antibody heavy chain variable region and/or an antibody light chain variable region, optionally an scFv, and is optionally bispecific or trispecific.

24. A modified immune cell for use according to any one of claims 1 to 16, wherein the antigen-specific receptor is a modified TCR that comprises:
a) a first antigen-binding chain comprising an antigen-binding fragment of a heavy chain variable region (VH) of an antibody; and
b) a second antigen-binding chain comprising an antigen-binding fragment of a light chain variable region (VL) of an antibody;
wherein the first and second antigen-binding chains each comprise a TRAC polypeptide or a TRBC polypeptide, optionally wherein at least one of the TRAC polypeptide and the TRBC polypeptide is endogenous, and optionally wherein one or both of the endogenous TRAC and TRBC polypeptides is inactivated.

25. A modified immune cell for use according to any one of claims 1 to 23 wherein a heterologous nucleic acid sequence encoding the antigen-specific receptor, or a portion thereof is inserted into the cell genome to express the antigen-specific receptor.

26. A modified immune cell for use according to any one of claims 1 to 23 wherein a heterologous nucleic acid sequence outside the cell genome expresses the antigen-specific receptor.

27. A modified immune cell for use according to claim 25, wherein insertion of the heterologous nucleic acid sequence encoding the antigen-specific receptor, or a portion thereof inactivates expression of a native TCR alpha chain and/or a native TCR beta chain.

28. A modified immune cell for use according to any of claims 1 to 27, wherein expression of the antigen-specific receptor is under control of an endogenous promoter of a TCR, optionally an endogenous TRAC promoter.

29. A modified immune cell for use according to any of claims 1 to 28, wherein the cell is a T cell, a CD4+ T cell, a CD8+ T cell, a CD4+ and CD8+ T cell, a NK cell, a T regulatory cell, a T_{N} cell, a memory stem T cell (T_{SCM}), a T_{CM} cell, a T_{EM} cell, a monocyte, a dendritic cell, or a macrophage, or a progenitor thereof, optionally a T cell progenitor, a lymphoid progenitor, an NK cell progenitor, a myeloid progenitor, a pluripotent stem cell, an induced pluripotent stem cell (iPSC), a hematopoietic stem cell (HSC), an adipose derived stem cell (ADSC), or a pluripotent stem cell of myeloid or lymphoid lineage.

30. A modified immune cell for use according to any of claims 1 to 29, wherein the immune cell is a T cell or NK cell, or progenitor thereof.

31. A modified immune cell for use according to any of claims 1 to 30, that further comprises a second engineered antigen-specific receptor, optionally a modified TCR or CAR, that specifically binds to a second antigen.

32. A modified immune cell for use according to any of claims 1 to 30, that comprises a first CAR that binds the antigen and a second CAR that binds a second antigen.

33. A modified immune cell for use according to any of claims 1 to 30, that comprises a CAR that binds the antigen and modified TCR that binds a second antigen.

34. A modified immune cell for use according to any of claims 1 to 30, that comprises a first modified TCR that binds the antigen and a second modified TCR that binds a second antigen.

35. A modified immune cell for use according to any of claims 1 to 30, that comprises three or more engineered antigen-specific receptors.

36. A modified immune cell for use according to any of claims 1 to 35 that further comprises a heterologous co-stimulatory receptor.

37. A modified immune cell for use according to claim 36, wherein the co-stimulatory receptor comprises (a) an extracellular domain of a co-stimulatory ligand, optionally from CD80, (b) a transmembrane domain, optionally from CD80, and (c) an intracellular domain of a co-stimulatory molecule, optionally CD28, 4-1BB, OX40, ICOS, DAP10, CD27, CD40, NKGD2, or CD2, preferably 4-1BB.

38. A modified immune cell for use according to any of claims 1 to 37, wherein the extracellular antigen-binding domain binds an antigen with a KD affinity of about 1 x 10⁻⁷ or less, about 5 × 10⁻⁸ or less, about 1 × 10⁻⁸ or less, about 5 × 10⁻⁹ or less, about 1 × 10⁻⁹ or less, about 5 × 10⁻¹⁰ or less, about 1 × 10⁻¹⁰ or less, about 5 × 10⁻¹¹ or less, about 1 × 10⁻¹¹ or less, about 5 × 10⁻¹² or less, or about 1 × 10⁻¹² or less.

39. A modified immune cell for use according to any of claims 1 to 38, wherein the antigen has a low density on the cell surface, of less than about 10,000, or less than about 5,000, or less than about 2,000 molecules per cell.

40. A modified immune cell for use according to any of claims 1 to 39, wherein SUV39H1 expression is reduced or inhibited by at least about 50%, 60%, 70%, 75%, 80%, 85%, 90% or 95%

41. A modified immune cell for use according to any of claims 1 to 40, wherein endogenous TCR expression is reduced by at least about 75%, 80%, 85%, 90% or 95%.

42. A modified immune cell for use according to any of claims 1 to 41 that is allogeneic.

43. A modified immune cell for use according to any of claims 1 to 41 that is autologous.

44. A modified immune cell for use according to any of claims 1 to 42, wherein the HLA-A locus is inactivated.

45. A modified immune cell for use according to claim 44, wherein HLA class I expression is reduced by at least about 75%, 80%, 85%, 90% or 95%.

46. A method of producing the cell of any of claims 1 to 45 comprising (a) introducing into the cell (a) a SUV39H1 inhibitor, and (b) (i) a nucleic acid encoding a CAR having one active ITAM or (ii) a heterologous nucleic acid encoding a portion of a modified TCR.

47. A modified immune cell for use according to any of claims 1 to 46, wherein the immune cell is a CAR T-cell and a dose of less than about 5 × 10⁷ cells, optionally about 10⁵ to about 10⁷ cells, is administered to the subject.

48. A modified immune cell for use according to any of claims 1 to 47, wherein a second therapeutic agent, optionally one or more cancer chemotherapeutic agents, cytotoxic agents, cancer vaccines, hormones, anti-angiogens, radiolabelled compounds, immunotherapy, surgery, cryotherapy, and/or radiotherapy, is administered to the subject

49. A modified immune cell for use according to any of claims 1 to 47, wherein the second therapeutic agent is an immune checkpoint modulator.

50. A modified immune cell for use according to claim 49, wherein the immune checkpoint modulator is an antibody that specifically binds to, or other inhibitor of, PD1, PDL1, CTLA4, LAG3, BTLA, OX2R, TIM-3, TIGIT, LAIR-1, PGE2 receptor, EP2/4 adenosine receptor, or A2AR, optionally an anti-PDl or anti-PDLl antibody.
